(19) Europäisches Patentamt
European Patent Office
Office européen des brevets

(11) Veröffentlichungsnummer : **0 242 716 B1**

# (12) EUROPÄISCHE PATENTSCHRIFT

(45) Veröffentlichungstag der Patentschrift : **20.03.91 Patentblatt 91/12**

(21) Anmeldenummer : **87105245.2**

(22) Anmeldetag : **09.04.87**

(51) Int. Cl.$^5$ : **C07D 213/40**, C07D 213/57, C07D 213/55, C07D 213/26, C07D 213/16, C09K 19/34, G02F 1/13

## (54) Flüssigkristalline Pyridinderivate.

Die Akte enthält technische Angaben, die nach dem Eingang der Anmeldung eingereicht wurden und die nicht in dieser Patentschrift enthalten sind.

(30) Priorität : **22.04.86 CH 1638/86**
**02.02.87 CH 356/87**

(43) Veröffentlichungstag der Anmeldung : **28.10.87 Patentblatt 87/44**

(45) Bekanntmachung des Hinweises auf die Patenterteilung : **20.03.91 Patentblatt 91/12**

(84) Benannte Vertragsstaaten : **CH DE FR GB IT LI SE**

(56) Entgegenhaltungen :
**DE-A- 3 404 055**
**DE-A- 3 524 489**

(73) Patentinhaber : **F. HOFFMANN-LA ROCHE AG**
**Postfach 3255**
**CH-4002 Basel (CH)**

(72) Erfinder : **Buchecker, Richard, Dr.**
**Felsenstrasse 10**
**CH-8008 Zürich (CH)**
Erfinder : **Germann, Alfred**
**Alemannengasse 109**
**CH-4058 Basel (CH)**
Erfinder : **Kelly, Stephen, Dr.**
**Violaweg 72**
**CH-4303 Kaiseraugst (CH)**
Erfinder : **Schadt, Martin, Dr.**
**Liestalerstrasse 77**
**CH-4411 Seltisberg (CH)**

(74) Vertreter : **Zimmermann, Hans, Dr. et al**
**Grenzacherstrasse 124 Postfach 3255**
**CH-4002 Basel (CH)**

**EP 0 242 716 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Pyridinderivate, flüssigkristalline Gemische, die solche Pyridinderivate enthalten, sowie die Verwendung für elektro-optische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können, Elektro-optische Vorrichtungen auf der Basis von Flüssigkristallen sind dem Fachmann gut bekannt und können auf verschiedenen Effekten beruhen, wie beispielsweise der dynamischen Streuung, der Deformation aufgerichteter Phasen (DAP-Zelle), dem Schadt-Helfrich-Effekt (Drehzelle), dem Gast/Wirt-Effekt (Guest/Host-Zelle) oder einem cholesterisch-nematischen Phasenübergang (Phase-Change-Zelle). Die meisten kommerziellen Anzeigevorrichtungen beruhen auf dem Schadt-Helfrich-Effekt mit verdrillt nematischer Struktur.

Die verwendeten Flüssigkristalle müssen eine gute chemische und thermische Stabilität und eine gute Stabilität gegenüber elektrischen Feldern und elektromagnetischer Strahlung aufweisen. Die Flüssigkristalle sollten ferner farblos sein, kurze Ansprechzeiten und niedere Viskositäten aufweisen, einen hohen Kontrast ergeben und bei üblichen Betriebstemperaturen eine geeignete Mesophase, beispielsweise eine nematische, cholesterische oder chirale smektische, vorzugsweise eine smektisch C, F oder I Phase besitzen. Da Flüssigkristalle üblicherweise als Mischungen zur Anwendung gelangen, ist es zudem wichtig, dass die Komponenten untereinander gut mischbar sind, Weitere Eigenschaften, wie beispielsweise die elektrische Leitfähigkeit, die Schwellenspannung, die dielektrische Anisotropie und die optische Anisotropie, müssen je nach verwendetem Zellentyp unterschiedliche Bedingungen erfüllen.

In letzter Zeit wurden in zunehmendem Masse Anzeigevorrichtungen mit hoher Informationsdichte (z.B. in Computerterminals) eingesetzt. Um die Zahl der benötigten Anschlüsse klein zu halten, wurde die Multiplexansteuerung weiterentwickelt. Hierzu werden jedoch Flüssigkristalle mit verbesserter Multiplexierbarkeit und somit steilen Transmissionskennlinien benötigt.

Die eingangs genannten Anzeigevorrichtungen besitzen im allgemeinen Ansprechzeiten in der Grössenordnung von mehreren Millisekunden oder höher.

Zur Verbesserung der Ansprechzeiten wurden in letzter Zeit auch Flüssigkristalle mit ferroelektrischen Eigenschaften eingesetzt. Bei dieser Anwendung werden chirale smektische Phasen, vorzugsweise smektisch C Phasen verwendet. Bisher sind jedoch nur wenige derartige Flüssigkristalle bekannt und die Mesophasebereiche sind meist zu eng oder liegen bei relativ hohen Temperaturen. Ferner ist die Stabilität dieser Flüssigkristalle oft ungenügend.

DE-A-35 24 489 offenbart u.a. 2-Phenylpyridine mit endständigen Alkyl-, Alkoxy- oder Cyanosubstituenten als nematische Flüssigkristallverbindungen und DE-A-34 04 055 offenbart 5-(4-Alkylphenyl oder 4-Alkoxyphenyl)-2-(4-cyanophenyl)pyridine als positiv dielektrische Materialien für nematische Flüssigkristalle. Bicyclische und tricyclische Verbindungen mit einem Pyridinring und endständigen Fluor-, Chlor-, Alkyl-, Alkoxy- oder Cyanosubstituenten werden ebenfalls in EP-A-0 194 153 als Komponenten für nematische Flüssigkristalle beschrieben.

Ferner offenbaren DE-A-37 01 629 und EP-A-0 228 303 u.a. 5-Alkyl-2-(4-alkoxyphenyl)pyridine mit chiralem Alkoxyrest und EP-A-0 233 706 offenbart u.a. entsprechende Verbindungen mit lateralem Fluor- oder Chlorsubstituenten in 3-Stellung des Phenylrings. Diese Verbindungen sind als Komponenten für chirale Flüssigkristalle, insbesondere für chirale smektische Phasen beschrieben.

Gegenstand der Erfindung sind 2,5-disubstituierte Pyridine der allgemeinen Formel

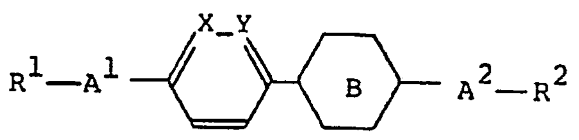

I

worin X für CH und Y für N steht oder X für N und Y für CH steht; Ring B trans-1,4-Cyclohexylen oder gegebenenfalls mit Halogen oder Methyl substituiertes 1,4-Phenylen darstellt; $A^1$ und $A^2$ einfache Kovalenzbindungen bedeuten oder eine der Gruppen $A^1$ und $A^2$ auch trans-1,4-Cyclohexylen oder gegebenenfalls mit Halogen oder Methyl substituiertes 1,4-Phenylen bedeutet; $R^1$ Alkyl oder Alkenyl bezeichnet, in welchen gegebenenfalls eine oder zwei nicht benachbarte $CH_2$-Gruppen durch Sauerstoff ersetzt sind, und $R^2$ Alkenyl bedeutet, in welchem gegebenenfalls eine oder zwei nicht benachbarte $CH_2$-Gruppen durch Sauerstoff ersetzt sind, oder $R^2$ an einem Benzolring auch –NCS bedeutet; oder $R^1$ Alkenyl bezeichnet, in welchem gegebenenfalls eine oder zwei nicht benachbarte $CH_2$-Gruppen durch Sauerstoff ersetzt sind, und $R^2$ Alkyl bedeutet, in welchem gegebenenfalls eine oder zwei nicht benachbarte $CH_2$-Gruppen durch Sauerstoff ersetzt sind, oder $R^2$ an einem

Benzolring auch Cyano oder Halogen bedeutet.

Die erfindungsgemässen Verbindungen besitzen die erforderliche gute Stabilität und sind farblos und gut mischbar mit andern Flüssigkristallen. Ferner besitzen die erfindungsgemässen Verbindungen kurze Ansprechzeiten, niedere Viskosität und ein verbessertes Mesophaseverhalten. Weiterhin ermöglichen die erfindungsgemässen Verbindungen tiefe Schwellenspannungen und aufgrund des günstigen Verhältnisses der elastischen Konstanten $k_{33}$ (bend) und $k_{11}$ (splay) sehr steile Transmissionskennlinien.

Die Verbindungen der Formel I, worin $R^2$ Cyano oder –NCS bedeutet, besitzen grosse positive Werte der dielektrischen Anisotropie. Die übrigen Verbindungen der Formel I besitzen kleine absolute Werte der dielektrischen Anisotropie.

Die Cyano- und NCS-Verbindungen mit geradkettiger Gruppe $R^1$ sowie die Verbindungen mit geradkettigen Gruppen $R^1$ und $R^2$ eignen sich vor allem als Komponenten für nematische Gemische sowie, in Kombination mit optisch aktiven Zusätzen, für cholesterische Gemische. Die Verbindungen der Formel I mit unpolaren Resten $R^1$ und $R^2$, insbesondere die optisch aktiven Verbindungen, welche in $R^1$ und/oder $R^2$ ein chirales Kohlenstoffatom aufweisen, eignen sich vor allem für Flüssigkristalle mit ferroelektrischen Eigenschaften. Die optische aktiven Verbindungen eignen sich aber ebenfalls als optisch aktive Zusätze für cholesterische Gemische.

Der Ausdruck "gegebenenfalls mit Halogen oder Methyl substituiertes 1,4-Phenylen" umfasst im Rahmen der vorliegenden Erfindung die Gruppen 1,4-Phenylen, 2-Fluor-1,4-phenylen, 2-Chlor-1,4-phenylen, 2-Brom-1,4-phenylen und 2-Methyl-1,4-phenylen. Der Ausdruck "Benzolring" bezieht sich auf diese Gruppen. Bevorzugt ist 1,4-Phenylen. Durch Verwendung substituierter Gruppen können aber gewünschtenfalls die Umwandlungstemperaturen, die Löslichkeit, die dielektrische Anisotropie und dergleichen modifiziert werden.

Der Ausdruck "Halogen" umfasst Fluor, Chlor oder Brom.

Der Ausdruck "Alkylgruppe, in welcher gegebenenfalls eine oder zwei nicht benachbarte $CH_2$-Gruppen durch –O– ersetzt sind", umfasst geradkettige und verzweigte Gruppen wie Alkyl, Alkoxy, Alkoxyalkyl, Alkoxyalkoxy und dergleichen.

Der Ausdruck "Alkenylgruppe, in welcher gegebenenfalls eine oder zwei nicht benachbarte $CH_2$-Gruppen durch –O– ersetzt sind", umfasst geradkettige und verzweigte Gruppen wie Alkenyl, Alkenyloxy, Alkoxyalkenyl, Alkenyloxyalkyl, Alkoxyalkenyloxy und dergleichen.

Eine bevorzugte Gruppe erfindungsgemässer Verbindungen sind die Verbindungen der Formel I, worin $R^2$ –NCS bedeutet, insbesondere die Verbindungen der allgemeinen Formel

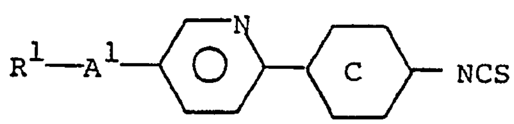

Ia

worin $R^1$ Alkyl oder Alkenyl bezeichnet, in welchen gegebenenfalls eine oder zwei nicht benachbarte $CH_2$-Gruppen durch Sauerstoff ersetzt sind ; $A^1$ eine einfache Kovalenzbindung, trans-1,4-Cyclohexylen oder gegebenenfalls mit Halogen oder Methyl substituiertes 1,4-Phenylen bedeutet; und Ring C gegebenenfalls mit Halogen oder Methyl substituiertes 1,4-Phenyen darstellt.

Diese Verbindungen besitzen vergleichsweise breite Mesophasebereiche und eine grosse positive dielektrische Anisotropie. Sie eignen sich insbesondere für Drehzellenanwendungen und ergeben steile Transmissionskennlinien und kurze Ansprechzeiten. $R^1$ ist vorzugsweise ein geradkettiger Rest.

Ferner sind Verbindungen der Formel I mit endständiger Cyanogruppe ($R^2$ = Cyano) bevorzugt, insbesondere diejenigen, worin $R^2$ für Cyano, $A^2$ für eine einfache Kovalenzbindung, Ring B für gegebenenfalls mit Halogen oder Methyl substituiertes 1,4-Phenylen, Y für N, X für CH und $A^1$ für eine einfache Kovalenzbindung, trans-1,4-Cyclohexylen oder gegebenenfalls mit Halogen oder Methyl substituiertes 1,4-Phenylen stehen. Vorzugsweise steht $R^1$ für die in Formel Ia genannten, ungesättigten Reste. Diese Verbindungen sind Zwischenprodukte für die Verbindungen der Formel I, worin $R^2$ –NCS bedeutet und $R^1$ eine C-C-Doppelbindung aufweist, und sind ebenfalls Flüssigkristalle mit grosser dielektrischer Anisotropie, günstigen elastischen Konstanten und verbessertem Mesophaseverhalten.

Ein weiterer bevorzugter Aspekt der vorliegenden Erfindung betrifft die Verbindungen der Formel I, worin $A^1$ und $A^2$ einfache Kovalenzbindungen bedeuten oder eine der Gruppen $A^1$ und $A^2$ auch trans-1,4-Cyclohexylen oder gegebenenfalls mit Halogen oder Methyl substituiertes 1,4-Phenylen bedeutet, einer der Reste $R^1$ und $R^2$ Alkenyl bezeichnet, in welchem gegebenenfalls eine oder zwei nicht benachbarte $CH_2$-Gruppen durch Sauerstoff ersetzt sind, und der andere der Reste $R^1$ und $R^2$ Alkyl oder Alkenyl bezeichnet, in welchen gegebenenfalls eine oder zwei nicht benachbarte $CH_2$-Gruppen durch Sauerstoff ersetzt sind. Diese Verbindungen besitzen kleine absolute Werte der dielektrischen Anisotropie Ferner besitzen sie vergleichsweise niedere Viskositäten und günstige elastische Konstanten und ermöglichen tiefe Schwellenspannungen. Die optisch inaktiven Verbindungen eignen sich insbesondere als unpolare Komponenten von nematischen oder cholestrischen Dielektrika für Drehzellen, Guest/Host-Zellen, Phase-Change-Zellen etc. Die optisch aktiven Verbindungen eignen sich insbesondere für ferroelektrische Anwendungen. Optische inaktive Verbindungen können aber, gewünschtenfalls auch in ferroelektrischen Gemischen verwendet werden. Ferner können optisch aktive Verbindungen auch als chirale Zusätze bei Drehzellenanwendungen, Phase-Change-Anwendungen und dergleichen verwendet werden.

Eine bevorzugte Gruppe erfindungsgemässer Verbindungen insbesondere für ferroelektrische Anwendungen sind die optisch aktiven Verbindungen der allgemeinen Formel

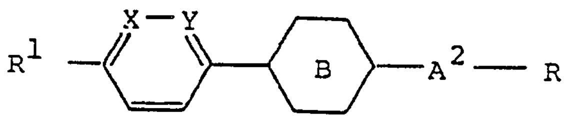

Ii

worin $R^1$, X, Y und Ring B die in Formel I gegebenen Bedeutungen haben ; $A^2$ eine einfache Kovalenzbindung, trans-1,4-Cyclohexylen oder gegebenenfalls mit Halogen oder Methyl substituiertes 1,4-Phenylen bedeutet ; und R eine Alkyl- oder Alkenylgruppe bezeichnet, in welcher gegebenenfalls eine oder zwei nicht benachbarte $CH_2$-Gruppen durch –O– ersetzt sind ; mit der Massgabe, dass $R^1$ und/oder R ein chirales Kohlenstoffatom aufweist.

Die Verbindungen der Formel Ii eignen sich insbesondere als Komponenten für Gemische mit ferroelektrischen Eigenschaften und besitzen zum grossen Teil selbst geeignete chirale smektische Phasen. Ring B in obiger Formel Ii steht vorzugsweise für gegebenenfalls mit Halogen oder Methyl substituiertes 1,4-Phenylen, insbesondere für 1,4-Phenylen. Die Tendenz zur Ausbildung chiraler smektischer Phasen wird durch die Anwesenheit einer Doppelbindung überraschenderweise im allgemeinen noch verstärkt. $R^1$ und/oder R in Formel Ii weist daher eine Doppelbindung auf. Formel Ii umfasst somit Verbindungen, worin einer der Reste $R^1$ und R Alkenyl bezeichnet, in welchem gegebenenfalls eine oder zwei nicht benachbarte $CH_2$-Gruppen durch Sauerstoff ersetzt sind und der andere der Reste $R^1$ und R Alkyl oder Alkenyl bezeichnet, in welchen gegebenenfalls eine oder zwei nicht benachbarte $CH_2$-Gruppen durch Sauerstoff ersetzt sind.

Der Formel Ii entsprechende optisch inaktive Verbindungen ohne chirales Kohlenstoffatom in $R^1$ und R aber mit mindestens einer C-C-Doppelbindung in $R^1$ und/oder R eignen sich ebenfalls als Komponenten ferroelektrischer Gemische. Vorzugsweise enthalten die Gemische in diesem Fall noch mindestens einen chiralen Zusatz.

Eine bevorzugte Gruppe unpolarer erfindungsgemässer Verbindungen insbesondere für nematische und cholesterische Anwendungen sind die Verbindungen der allgemeinen Formel

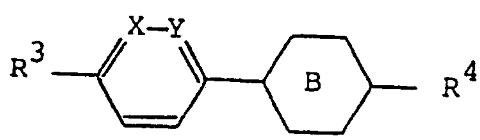

Ib

worin einer der Reste $R^3$ und $R^4$ eine Alkenylgruppe bezeichnet, in welcher gegebenenfalls eine oder zwei nicht benachbarte $CH_2$-Gruppen durch –O– ersetzt sind ; der andere der Reste $R^3$ und $R^4$ eine Alkyl- oder Alkenylgruppe bedeutet, in welcher gegebenenfalls eine oder zwei nicht benachbarte $CH_2$-Gruppen durch –O– ersetzt sind ; und X, Y und Ring B die in Formel I gegebenen Bedeutungen haben.

Die Verbindungen der Formel Ib sind niederviskose Verbindungen mit besonders günstigen elastischen Konstanten und ermöglichen trotz kleinen absoluten Werten der dielektrischen Anisotropie tiefe Schwellenspannungen Ring B in Formel Ib stellt trans-1,4-Cyclohexylen oder gegebenenfalls mit Halogen oder Methyl substituiertes 1,4-Phenylen dar.

Die Variierung der Stellung der Doppelbindung in Formel Ib erlaubt eine weitere Optimierung der elektrooptischen und mesomorphen Eigenschaften. Beispielsweise besitzen die Verbindungen, worin $R^3$ und/oder $R^4$ 1E-Alkenyl, 2E-Alkenyloxy, 3E-Alkenyl, 4-Alkenyloxy oder 5-Alkenyl bedeutet, besonders günstige Mesophasebereiche und kurze Ansprechzeiten. Anderseits besitzen beispielsweise die Verbindungen der Formel Ib, worin $R^3$ und/oder $R^4$ 2Z-Alkenyl, 3-Alkenyloxy oder 4-Alkenyl bedeutet, besonders niedrige Verhältnisse der elastischen Konstanten $k_{33}$ und $k_{11}$ und ergeben besonders tiefe Schwellenspannungen.

Die tricyclischen Verbindungen, d.h. die Verbindungen der Formel I, worin $A^1$ oder $A^2$ trans-1,4-Cyclohe-

xylen, oder gegebenenfalls mit Halogen oder Methyl substituiertes 1,4-Phenylen bedeutet, besitzen hohe Klärpunkte und ergeben ebenfalls steile Transmissionskennlinien.

In obigen Formeln I, Ii und Ib steht vorzugsweise X für CH und Y für N. Ring B in den Formeln I und Ib bezeichnet vorzugsweise trans-1,4-Cyclohexylen oder 1,4-Phenylen und Ring C in Formel Ia bezeichnet vorzugsweise 1,4-Phenylen.

Bevorzugte Alkyl- und Alkenylgruppen $R^1$, $R^2$, $R^3$, $R^4$ und R, in welchen gegebenenfalls eine oder zwei nicht benachbarte $CH_2$-Gruppen durch –O– ersetzt sind, sind die oben genannten Gruppen.

Bevorzugte Alkyl- und Alkenylgruppen, in denen gegebenenfalls eine oder zwei nicht benachbarte $CH_2$-Gruppen durch Sauerstoff ersetzt sind, sind Alkyl, Alkoxy, Alkoxyalkyl, Alkoxyalkoxy, Alkenyl, Alkenyloxy, Alkoxyalkenyl, Alkoxyalkenyloxy und Alkenyloxyalkyl insbesondere Alkyl, Alkoxy, Alkenyl und Alkenyloxy. Vorzugsweise steht eine in $R^1$ und/oder $R^2$ gegebenenfalls vorhandene Doppelbindung in Stellung 1, 3 oder 4 (unter Einschluss allfällig vorhandener Sauerstoffatome) der Seitenkette $R^1$ bzw. $R^2$, insbesondere in Stellung 4. Besonders bevorzugte ungesättigte Gruppen sind somit Gruppen wie 1E-Alkenyl, 3E-Alkenyl, 4-Alkenyl, 2E-Alkenyloxy, 3-Alkenyloxy, Alkoxy-3E-alkenyl, Alkoxy-4-alkenyl, Alkoxy-3-alkenyloxy und dergleichen. Vorzugsweise stehen daher $R^1$ und/oder $R^2$ in Formel I, $R^1$ in Formel Ia, $R^1$ und/oder R in Formel Ii und $R^3$ und/oder $R^4$ in Formel Ib für die genannten Gruppen.

Die Reste $R^1$ und $R^2$ weisen höchstens je 18 Kohlenstoffatome auf. Geradkettige Gruppen $R^1$ bzw. $R^2$ weisen bevorzugt je 1-12 Kohlenstoffatome und besonders bevorzugt je 1-7 Kohlenstoffatome auf. Gruppen $R^1$ bzw. $R^2$ mit chiralen Kohlenstoffatomen besitzen vorzugsweise 4-18 Kohlenstoffatome und besonders bevorzugt 4-15 Kohlenstoffatome.

Optisch aktive Verbindungen der Formel I bzw. Ii für ferroelektrische Anwendungen enthalten in $R^1$ und $R^2$ zusammen im Falle bicyclischer Verbindungen vorzugsweise mindestens 10, insbesondere mindestens 12 Kohlenstoffatome und im Falle tricyclischer Verbindungen vorzugsweise mindestens 8, insbesondere mindestens 10 Kohlenstoffatome. Analoges gilt auch bei Verwendung achiraler Verbindungen für ferroelektrische Gemische. Als chirale Dotierstoffe für cholesterische oder chiral smektische Gemische sind aber auch Verbindungen mit geringerer Kohlenstoffzahl in $R^1$ und $R^2$ geeignet.

Bevorzugte Gruppen mit chiralem Kohlenstoffatom sind die Gruppen der allgemeinen Formel

$$-(Z^1)_p-C_nH_{2n}-\overset{\overset{\displaystyle R^5}{|}}{\underset{\underset{\displaystyle R^6}{|}}{C^*}}-H \qquad \text{IV}$$

$$-(Z^1)_p-C_mH_{2m-2}-\overset{\overset{\displaystyle R^7}{|}}{\underset{\underset{\displaystyle R^8}{|}}{C^*}}-H \qquad \text{V}$$

worin m, n und p ganze Zahlen bedeuten und p für 0 oder 1, n für 0-6 und m für 2-6 stehen ; $R^5$ Alkyl und $R^6$ Alkoxy, Alkenyl, Alkenyloxy oder von $R^5$ verschiedenes Alkyl bedeuten ; oder $R^5$ Alkenyl und $R^6$ Alkoxy bedeuten ; $R^7$ Alkyl und $R^8$ Alkoxy oder von $R^7$ verschiedenes Alkyl bezeichnen ; und $Z^1$ die Gruppe $-CH_2$-oder –O– darstellt.

In den obigen Formeln IV und V steht vorzugsweise $Z^1$ für Sauerstoff. Besonders bevorzugte chirale Reste sind die Gruppen der Formel IV und V, worin $R^5$ bzw. $R^7$ Methyl und $R^6$ bzw. $R^8$ von Methyl verschiedenes Alkyl, insbesondere Aethyl bezeichnen.

Besonders bevorzugte optisch aktive Verbindungen der Formel I bzw. Ii sind diejenigen, worin einer der Reste $R^1$ und $R^2$ bzw. einer der Reste $R^1$ und R eine chirale Gruppe der Formel IV oder V bezeichnet und der andere der Reste $R^1$ und $R^2$ bzw. der andere der Reste $R^1$ und R eine geradkettige Alkyl-, Alkoxy-, Alkenyl- oder Alkenyloxygruppe oder eine chirale Gruppe der Formel IV oder V bedeutet.

Die Verbindungen der Formel I können nach an sich bekannten Methoden hergestellt werden, beispielsweise nach den in den Schemata 1-3 illustrierten Methoden, worin $A^1$, $A^2$, X, Y und Ring B die in Formel I gegebenen Bedeutungen haben, Ts p-Tosyl bedeutet, $R^1$ und $R^{11}$ Alkyl- oder Alkenylgruppen bezeichnen, in welchen gegebenenfalls eine oder zwei nicht benachbarte $CH_2$-Gruppen durch Sauerstoff ersetzt sind, und $R^9$ und $R^{10}$ Alkyl bedeuten, in welchem gegebenenfalls eine oder zwei nicht benachbarte $CH_2$-Gruppen durch Sauerstoff ersetzt sind, oder $R^{10}$ auch Halogen bedeutet.

Schema 1

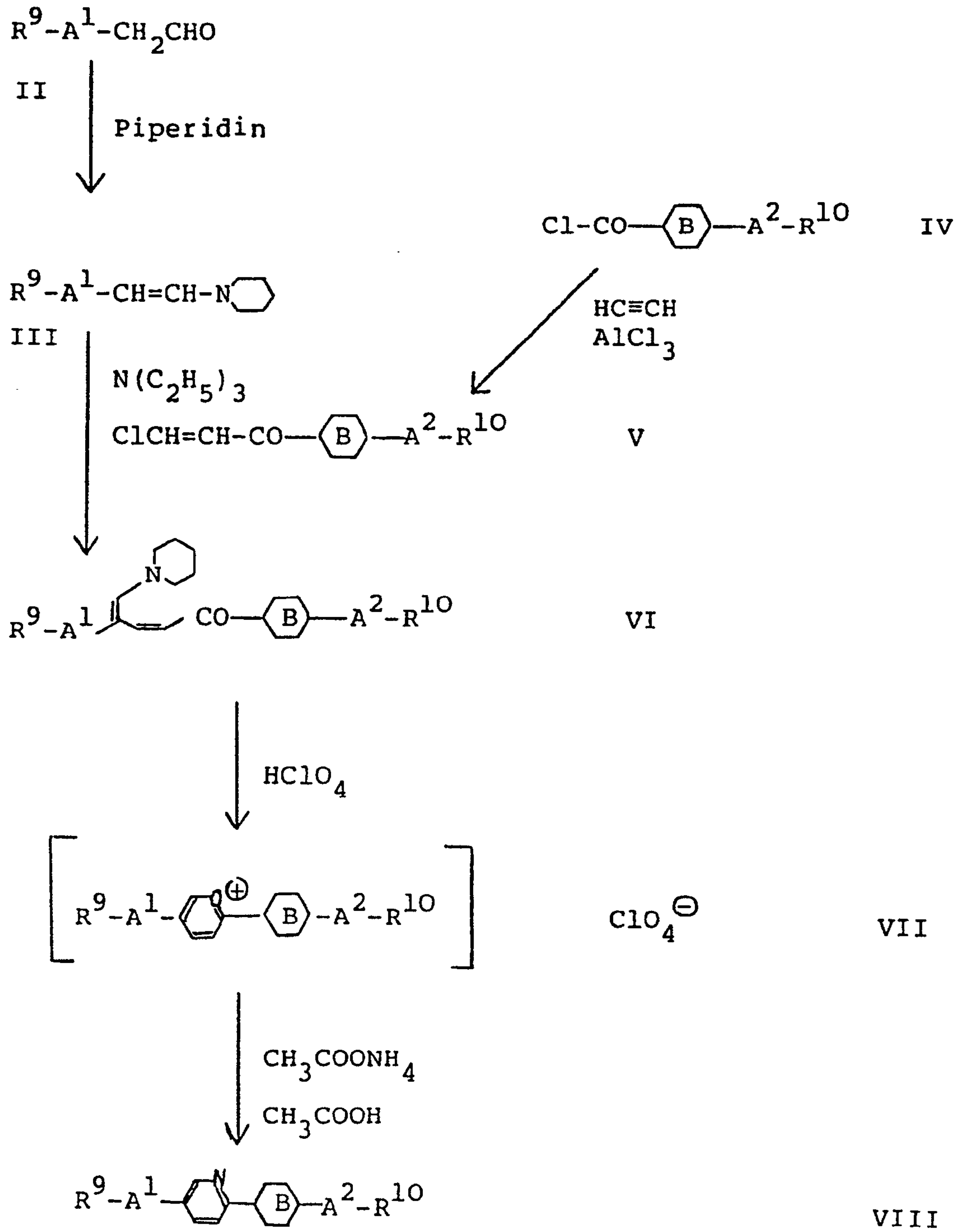
II
Piperidin
IV
HC≡CH
AlCl3
III
N(C2H5)3
V
VI
HClO4
VII
CH3COONH4
CH3COOH
VIII

Schema 2

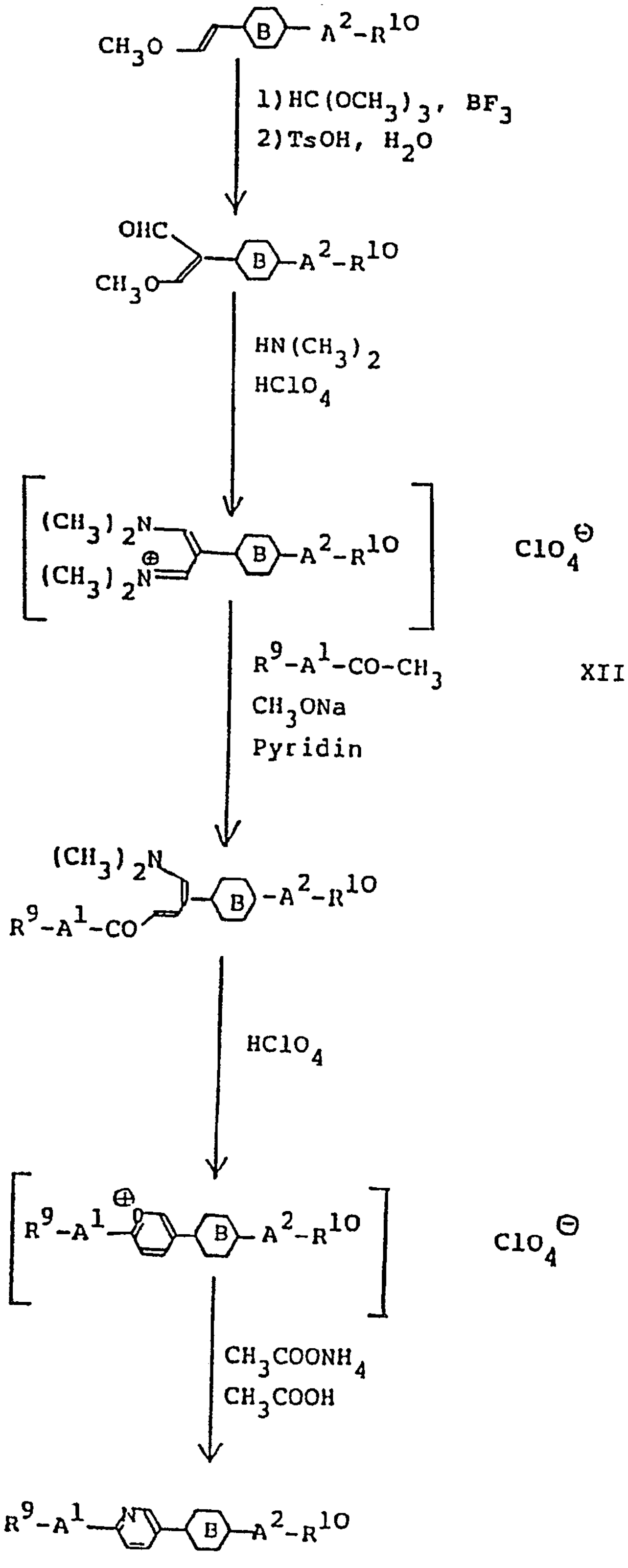


IX

X

XI

XII

XIII

XIV

XV

Schema 3

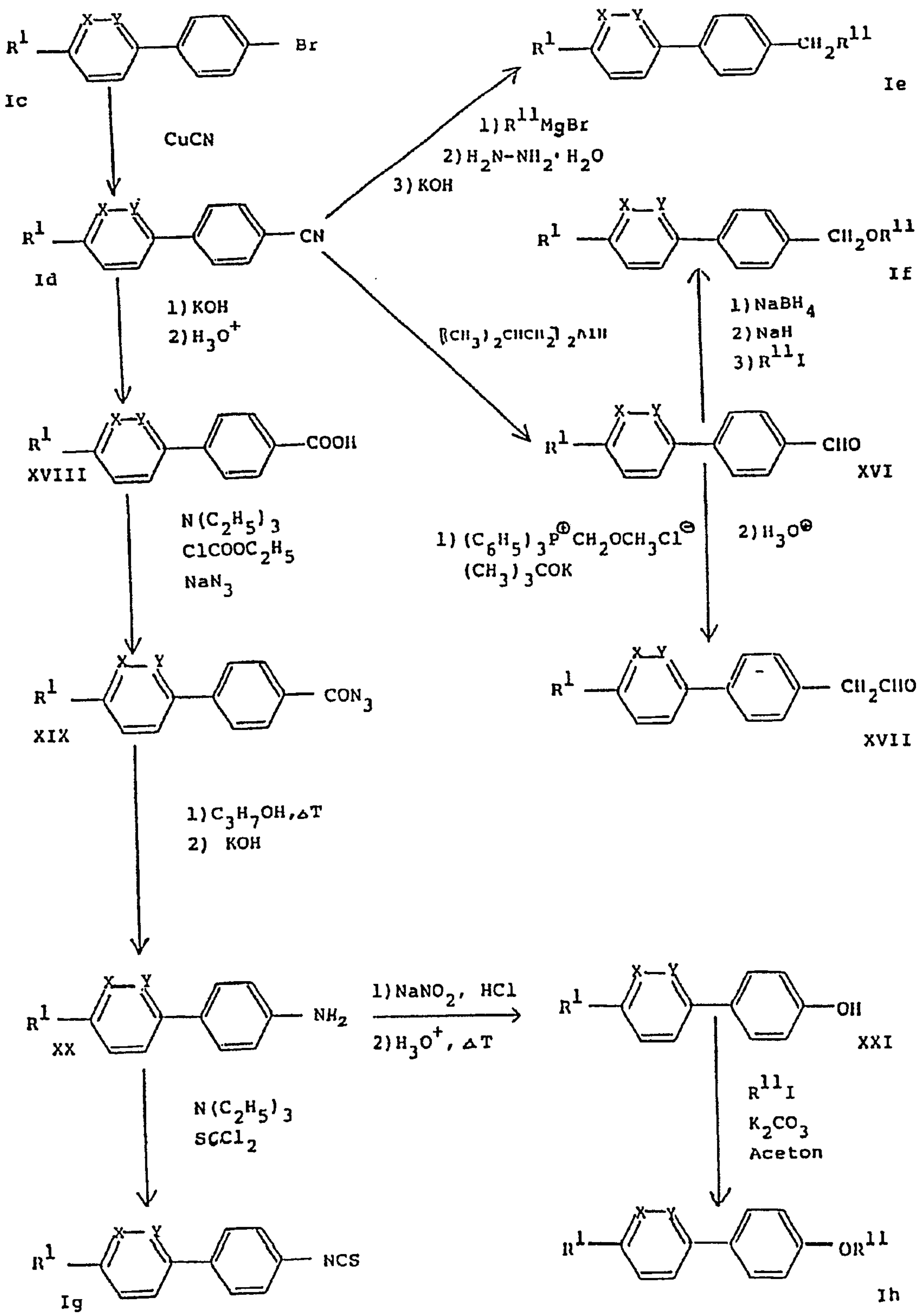
Ic
CuCN
Id
1) R11MgBr
2) H2N-NH2 · H2O
3) KOH
Ie
If
1) NaBH4
2) NaH
3) R11I
[(CH3)2CHCH2]2AlH
1) KOH
2) H3O+
XVIII
XVI
N(C2H5)3
ClCOOC2H5
NaN3
1) (C6H5)3P⊕CH2OCH3Cl⊖
(CH3)3COK
2) H3O⊕
XIX
XVII
1) C3H7OH, ΔT
2) KOH
XX
1) NaNO2, HCl
2) H3O+, ΔT
XXI
N(C2H5)3
SCCl2
R11I
K2CO3
Aceton
Ig
Ih

Die Ausgangsmaterialien der Formeln II, IV, IX und XII sind bekannte oder Analoge bekannter Verbindungen und können nach bekannten Methoden hergestellt werden, beispielsweise aus den entsprechenden Nitrilen. Die Aldehyde der Formel II können beispielsweise aus den entsprechenden oder homologen Nitrilen erhalten werden durch Umsetzung mit Diisobutylaluminiumhydrid und gegebenenfalls anschliessende Ueberführung in homologe Aldehyde durch Umsetzung mit Methoxymethyl-triphenylphosphoniumchlorid und Hydrolyse des erhaltenen Enoläthers mit Säure. Die Säurechloride der Formel IV können beispielsweise aus den entsprechenden Nitrilen erhalten werden durch Hydrolyse und anschliessende Umsetzung der erhaltenen Carbonsäure mit Thionylchlorid, Die Methoxyvinyl-Verbindungen der Formel IX können beispielsweise aus den entsprechenden Nitrilen erhalten werden durch Reduktion mit Diisobutylaluminiumhydrid und anschliessende Umsetzung der erhaltenen Aldehyde mit Methoxyvinyl-triphenylphosphoniumchlorid. Die Methylketone der Formel XII können beispielsweise aus den entsprechenden Nitrilen erhalten werden durch Umsetzung mit Methylmagnesiumbromid.

In analoger Weise zu den in Schema 3 illustrierten Methoden können auch die tricyclischen Verbindungen der Formel I hergestellt werden.

Zur Herstellung der Verbindungen der Formel I, welche in $R^1$ und/oder $R^2$ eine C-C-Doppelbindung aufweisen, werden anstelle der den Formeln II, IV, IX und XII entsprechenden ungesättigten Verbindungen zweckmässigerweise solche Aldehyde, Säurechloride, Methoxyvinyl-Verbindungen bzw. Methylketone als Ausgangsmaterialien eingesetzt, welche anstelle der Doppelbindung die Gruppe >CBr-CBr< oder eine geschützte Hydroxymethylengruppe, z.B. die Gruppe $CH_3COOCH<$ oder $p\text{-}CH_3O\text{-}C_6H_4\text{-}OCH<$ aufweisen.

Die benötigten bromierten Verbindungen, d.h. die den Formeln II, IV, IX und XII entsprechenden Verbindungen, welche in $R^9$ und $R^{10}$ zwei vicinale Bromsubstituenten aufweisen, können in analoger Weise zu den oben beschriebenen Methoden aus den entsprechenden bromierten Nitrilen hergestellt werden. Die bromierten Nitrile können aus den entsprechenden ungesättigten Nitrilen erhalten z.B. durch Umsetzung mit Brom in Chloroform.

Die benötigten Verbindungen mit geschützter Hydroxymethylgruppe können ebenfalls nach an sich bekannten Methoden hergestellt werden. Die benötigten Acetate können beispielsweise aus den entsprechenden Cyanoaldehyden erhalten werden, z.B. durch Reduktion der Formylgruppe zur Hydroxymethylgruppe mittels Natriumborhydrid oder katalytischer Hydrierung, anschliessende Umsetzung der Nitrilgruppe in analoger Weise zu den oben angegebenen Methoden und Acetylierung der Hydroxymethylgruppe mit Acetylchlorid oder Acetanhydrid. Die p-Methoxyphenolate können beispielsweise aus den entsprechenden Cyanoaldehyden erhalten werden, z.B. durch Reduktion der Formylgruppe zur Hydroxymethylgruppe mittels Natriumborhydrid oder katalytischer Hydrierung, anschliessende Verätherung der Hydroxymethylgruppe mit p-Methoxyphenol nach der in Tetrahedron Letters 26, 2691 (1985) beschriebenen Methode und Umsetzung der Nitrilgruppe in analoger Weise zu den oben angegebenen Methoden.

Die Ueberführung der Dibromide in ungesättigte Verbindungen erfolgt zweckmässig nach dem Aufbau des Pyridinrings, d.h. an den Dibromiden entsprechend den Formeln VIII und XV. Die Umsetzung kann in an sich bekannter Weise, beispielsweise mit Zink in Eisessig erfolgen.

Die Ueberführung der geschützten Hydroxymethylgruppe in eine Gruppe mit einer C-C-Doppelbindung erfolgt ebenfalls zweckmässig nach dem Aufbau des Pyridinrings, wobei die Acetatgruppe bereits bei der Umsetzung mit Perchlorsäure hydrolysiert werden kann. Die Ueberführung kann nach an sich bekannten Methoden erfolgen, beispielsweise durch Hydrolyse der Acetatgruppe bzw. durch oxidative Entfernung der p-Methoxyphenolatgruppe mit Cer-(IV)-ammoniumnitrat in Acetonitril/Wasser, Ueberführung der Hydroxymethylgruppe in die Formylgruppe mit Oxalylchlorid in Dimethylsulfoxid und Umsetzung der erhaltenen Aldehyde mit Wittig-Reagenzien, z.B. mit Alkyl-triphenylphosphoniumbromid. Gewünschtenfalls können die Aldehyde vor der Wittig-Reaktion in homologe Aldehyde übergeführt werden durch Umsetzung mit Methoxymethyl-triphenylphosphoniumchlorid und anschliessende Hydrolyse des Enoläthers mit Säure.

Die nach Abspaltung der Schutzgruppe erhaltenen Zwischenprodukte mit einer Hydroxymethylgruppe können gewünschtenfalls auch in an sich bekannter Weise mit Alkyljodid, Alkenyljodid etc. veräthert werden zu Verbindungen der Formel I, worin $R^1$ und/oder $R^2$ eine Gruppe –O– aufweist.

Verbindungen der Formel I mit einer C-C-Doppelbindung in $R^2$ können auch nach den in Schema 3 illustrierten Methoden erhalten werden.

Die Aldehyde der Formel XVII können gewünschtenfalls durch sukzessive Kettenverlängerung in weitere homologe Aldehyde übergeführt werden. Verbindungen XVI und XVII und Homologe dieser Verbindungen können durch Umsetzung mit Wittig-Reagenzien, z.B. mit Alkyl-triphenylphosphoniumbromid in Verbindungen der Formel I übergeführt werden, welche in $R^2$ eine C-C-Doppelbindung aufweisen, oder mit Natriumborhydrid reduziert und die erhaltenen Alkohole veräthert werden (analog zur Reaktion XVI→If).

Die erfindungsgemässen Verbindungen können in Form von Gemischen untereinander und/oder mit anderen Flüssigkristallkomponenten verwendet werden, wie z.B. mit Substanzen aus den Klassen der Schiffschen

Basen, Azobenzole, Azoxybenzole, Phenylbenzoate, Cyclohexancarbonsäurephenylester, Cyclohexancarbonsäurecyclohexylester, Biphenyle, Phenylcyclohexane, Phenylpyrimidine, Cyclohexylpyrimidine, Phenyldioxane, 2-Cyclohexyl-1-phenyläthane und dergleichen. Derartige Substanzen sind dem Fachmann bekannt und viele davon sind zudem im Handel erhältlich.

Aufgrund der günstigen Eigenschaften der erfindungsgemässen Verbindungen kann ihr Anteil in flüssigkristallinen Gemischen in breiten Grenzen variieren und bis zu 100% betragen. Im allgemeinen enthalten die erfindungsgemässen Mischungen etwa 1-80 Gew.-%, vorzugsweise etwa 5-50 Gew.-% an Verbindungen der Formel I. Bei Verwendung chiraler Verbindungen der Formel I als optisch aktive Zusätze zur Herstellung cholesterischer Gemische kann der Anteil auch geringer sein und je nach gewünschter Ganghöhe (pitch) beispielsweise etwa 0,2-50 Gew.-% betragen.

Die erfindungsgemässen Flüssigkristallmischungen mit nematischen oder cholesterischen Eigenschaften enthalten neben einer oder mehreren Verbindungen der Formel I vorzugsweise eine oder mehrere der Verbindungen der folgenden allgemeinen Formeln

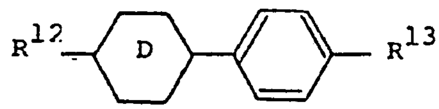
XXII

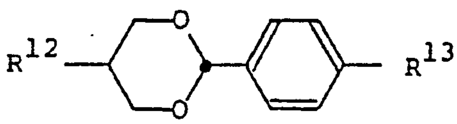
XXIII

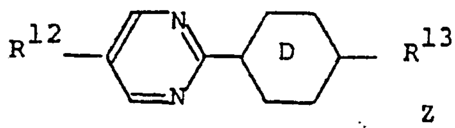
XXIV

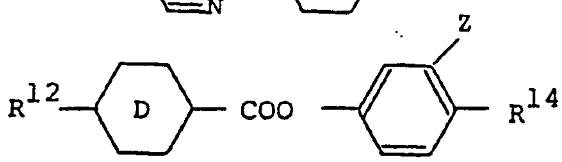
XXV

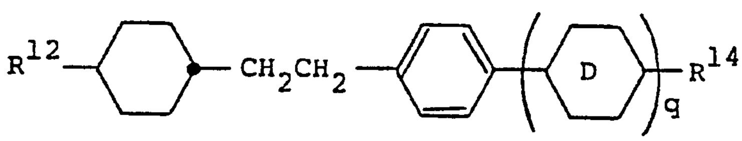
XXVI

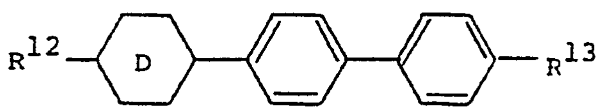
XXVII

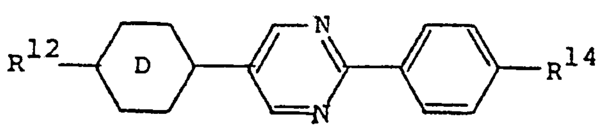
XXVIII

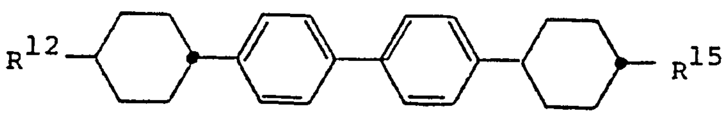
XXIX

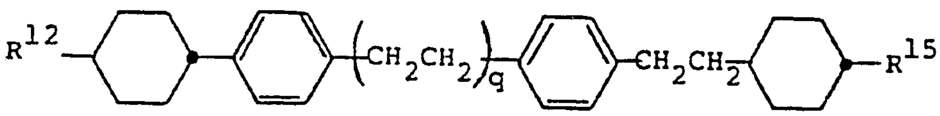
XXX

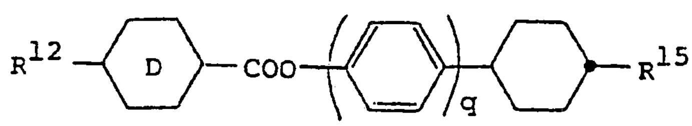
XXXII

worin $R^{12}$, $R^{13}$, $R^{14}$ und $R^{15}$ unabhängig voneinander Alkyl, Alkoxy, Alkenyl oder Alkenyloxy mit 1 bis 7 Kohlenstoffatomen bedeuten oder $R^{13}$ an einem Benzolring auch Cyano oder –NCS bedeutet oder $R^{14}$ an einem Benzolring auch Cyano bedeutet ; Ring D trans-1,4-Cyclohexylen oder 1,4-Phenylen darstellt ; Z Wasserstoff oder Fluor bezeichnet ; und q für die Zahl 0 oder 1 steht.

Die erfindungsgemässen Flüssigkristallmischungen mit ferroelektrischen Eigenschaften enthalten neben einer oder mehreren Verbindungen der Formel I, worin $R^2$ eine gegebenenfalls halogensubstituierte Alkyl- oder

Alkenylgruppe bedeutet, in welcher gegebenenfalls eine oder zwei nicht benachbarte $CH_2$-Gruppen durch –O–, –COO– und/oder –OOC– ersetzt sind, vorzugsweise eine oder mehrere Verbindungen der allgemeinen Formeln

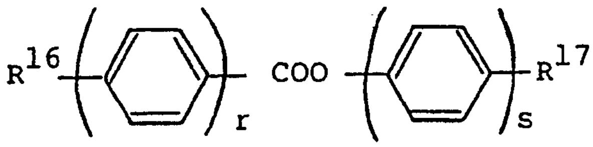

XXXI

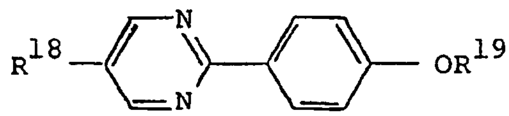

XXXIII

worin $R^{16}$ und $R^{17}$ Alkyl, Alkoxy, Alkanoyl, Alkanoyloxy, Alkoxycarbonyl oder Alkoxycarbonyloxy bedeuten ; $R^{18}$ und $R^{19}$ Alkyl darstellen ; und r und s die Zahlen 1 oder 2 bezeichnen.

Vorzugsweise sind eine oder mehrere Komponenten des ferroelektrischen Gemisches optisch aktiv. Bevorzugt ist daher die Verwendung optisch aktiver Verbindungen der Formel I mit chiralen Kohlenstoffatom in $R^1$ und/oder $R^2$. Bevorzugte Zusätze sind die optisch aktiven Verbindungen der Formel XXXI und XXXIII, worin $R^{16}$ oder $R^{17}$ bzw. $R^{18}$ oder $R^{19}$ ein chirales Kohlenstoffatom aufweist.

Die erfindungsgemässen Mischungen können ebenfalls dichroitische Farbstoffe, beispielsweise Azo-, Azoxy- oder Anthrachinonfarbstoffe, enthalten. Der Anteil der Farbstoffe wird durch die Löslichkeit und die gewünschte Farbe, Extinktion und dergleichen bestimmt und beträgt im allgemeinen höchstens etwa 10 Gew.-% im Gesamtgemisch.

Die Herstellung der erfindungsgemässen Mischungen kann in an sich bekannter Weise erfolgen, z.B. durch Erhitzen einer Mischung der Bestandteile auf eine Temperatur knapp oberhalb des Klärpunktes und anschliessendes Abkühlen. Die Herstellung einer elektro-optischen Vorrichtung kann ebenfalls in an sich bekannter Weise erfolgen, z.B. durch Evakuieren einer geeigneten Zelle und Einbringen der Mischung in die evakuierte Zelle.

Die Herstellung der erfindungsgemässen Verbindungen und Gemische wird durch die folgenden Beispiele weiter veranschaulicht. Die Phasen werden durch folgende Symbole bezeichnet : C für kristallin, S für smektisch, $S_A$ für smektisch A, $S_C$ für smektisch C, $S_c^*$ für chiral smektisch C, N für nematisch, Ch für cholesterisch und I für isotrop. $p_o = (V_{50}-V_{10})/V_{10}$ ist ein Mass für die Steilheit der Transmissionskennlinie, wobei $V_{10}$ und $V_{50}$ die Spannung für 10% bzw. 50% Transmission (in einer Drehzelle bei 0° Kippwinkel) bedeuten. $\Delta n$ bezeichnet die optische Anisotropie.

Beispiel 1

a) Ein Gemisch von 3,026 g 2-(p-Cyanophenyl)-5-pentylpyridin, 125 ml Diäthylenglykol und 13,8 g Kaliumhydroxid wurde unter Rühren und Stickstoffbegasung 1,5 Stunden auf 175°C erhitzt. Dann wurde das Reaktionsgemisch abkühlen gelassen, mit halbkonzentrierter Salzsäure auf pH 8 gestellt und mit Methylenchlorid extrahiert. Die organische Phase wurde mit Wasser gewaschen, über Natriumsulfat getrocknet, filtriert und eingeengt. Die erhaltene, rohe p-(5-Pentyl-2-pyridyl)benzoesäure wurde aus Aceton/Hexan umkristallisiert.

b) Eine Lösung von 2,1 g p-(5-Pentyl-2-pyridyl)benzoesäure in 80 ml Aceton wurde unter Rühren und Stickstoffbegasung auf 2°C gekühlt und innert 10 Minuten tropfenweise mit einer Lösung von 2,16 ml Triäthylamin in 15 ml Aceton versetzt. Dann wurde das Reaktionsgemisch bei 1-2°C innert 10 Minuten tropfenweise mit einer Lösung von 1,86 ml Chlorameisensäureäthylester in 8 ml Aceton versetzt und noch 30 Minuten bei dieser Temperatur gerührt. Anschliessend wurde bei 1-2°C eine Lösung von 1,38 g Natriumazid in 8 ml Wasser innert 10 Minuten zum Reaktionsgemisch zugetropft und noch 1 Stunde in Eisbad gerührt. Danach wurde das Reaktionsgemisch auf 200 ml Wasser gegossen und mit Diäthyläther extrahiert. Die organische Phase wurde mit Wasser gewaschen, über Natriumsulfat getrocknet, filtriert und vom Lösungsmittel befreit. Chromatographische Reinigung der erhaltenen, leicht gelblichen Kristalle (2,1 g) an 120 g Kieselgel mit 1 l Methylenchlorid ergab 2,15 g p-(5-Pentyl-2-pyridyl)benzoesäureazid als leicht gelbliche Paste.

c) 2,147 g p-(5-Pentyl-2-pyridyl)benzoesäureazid wurden unter Stickstoff in 60 ml n-Propanol gelöst und die Lösung 45 Minuten bei 140°C gerührt. Nach Abdampfen des Lösungsmittels am Rotationsverdampfer wurden 2,24 g Propyl-p-(5-pentyl-2-pyridyl)phenylcarbamat als leicht gelbliche Kristalle erhalten.

d) eine Lösung von 2,24 g Propyl-p-(5-pentyl-2-pyridyl)phenylcarbamat in 186 ml Diäthylenglykol wurde mit 26 g Kaliumhydroxid und 47 ml Wasser versetzt und unter Stickstoff auf 170°C erhitzt, Nach 1,75 Stunden wurde das Reaktionsgemisch abgekühlt, in 250 ml Wasser gegossen und mit Methylenchlorid extrahiert. Die organische Phase wurde über Natriumsulfat getrocknet, filtriert und am Rotationsverdampfer unter Vakuum vom Lösungsmittel befreit. Chromatographische Reinigung des Rückstandes (1,39 g) an 60 g Kieselgel mit 1,5 l Methylenchlorid und 0,5 l Methylenchlorid/Aethylacetat (Vol. 4 : 1) und anschliessende Umkristallisation aus Methylenchlorid/Hexan ergab 0,731 g p-(5-Pentyl-2-pyridyl)anilin als gelbliche Kristalle ; Smp. 60,3-61,3°C.

e) Eine Lösung von 1,256 g p-(5-Pentyl-2-pyridyl)anilin in 29 ml Chloroform wurde bei 5°C unter Stickstoff mit 1,46 ml Triäthylamin versetzt. Dann wurde bei 1°C innert 20 Minuten eine Lösung von 0,49 ml 95-proz. Thiophosgen in 14,5 ml Chloroform zum Reaktionsgemisch so zugetropft, dass die Temperatur 3°C nicht überstieg. Anschliessend wurde das Reaktionsgemisch 45 Minuten bei Raumtemperatur und weitere 40 Minuten unter Rückfluss gerührt, dann abgekühlt und mit 40 ml 3N Ammoniak und mit Wasser gewaschen. Die wässrigen Phasen wurden mit Methylenchlorid nachextrahiert. Die organischen Phasen wurden über Natriumsulfat getrocknet, filtriert und vom Lösungsmittel befreit. Chromatographische Reinigung der erhaltenen bräunlichen Paste (1,56 g) an 60 g Kieselgel mit 700 ml Methylenchlorid/Hexan (Vol. 1 : 1) und anschliessende Kristallisation aus Diäthyläther/Hexan ergab 1,17 g p-(5-Pentyl-2-pyridyl)phenylisothiocyanat als farblose Kristalle mit Smp. (C-$S_A$) 36,3°C und Klp. ($S_A$-I) 97,2°C.

In analoger Weise können folgende Verbindungen hergestellt werden :

p-(5-Propyl-2-pyridyl)phenylisothiocyanat,
p-(5-Hexyl-2-pyridyl)phenylisothiocyanat ; Smp. (C-$S_A$) 12,6°C, Klp. ($S_A$-I) 99,0°C,
p-(5-Heptyl-2-pyridyl)phenylisothiocyanat,
p-[5-(3-Butenyl)-2-pyridyl]phenylisothiocyanat,
p-[5-(3E-Pentenyl)-2-pyridyl]phenylisothiocyanat,
p-[5-(4-Pentenyl)-2-pyridyl]phenylisothiocyanat,
p-[5-(4-Aethylphenyl)-2-pyridyl]phenylisothiocyanat,
p-[5-(4-Pentylphenyl)-2-pyridyl]phenylisothiocyanat,
p-[5-(4-(4-Pentenyl)phenyl)-2-pyridyl]phenylisothiocyanat,
p-[5-(trans-4-Aethylcyclohexyl)-2-pyridyl]phenylisothiocyanat,
p-[5-(trans-4-pentylcyclohexyl)-2-pyridyl]phenylisothiocyanat,
p-[5-(trans-4-(4-Pentenyl)cyclohexyl)-2-pyridyl]phenylisothiocyanat.

Beispiel 2

Eine Suspension von 5,59 g 2-(p-Bromphenyl)-5-(4-pentenyl)pyridin, 4 ml Dimethylformamid und 1,98 g Kupfer-(I)-cyanid wurde unter Rühren 3 Stunden auf 180°C erhitzt. Dann wurde das braune Reaktionsgemisch auf Raumtemperatur abgekühlt, anschliessend auf eine Mischung von 3,67 g Eisen-(III)-chlorid, 36,7 ml Wasser und 0,245 ml konzentrierter Salzsäure gegossen und noch 1 Stunde bei 55°C gerührt. Danach wurde das Gemisch abgekühlt und zwischen Methylenchlorid und Wasser verteilt. Die organische Phase wurde mit Wasser gewaschen, über Natriumsulfat getrocknet und filtriert. Das Filtrat wurde eingedampft und der Rückstand an 250 g Kieselgel mit 2,5 l Methylenchlorid unter leichtem Druck chromatographisch gereinigt. Das erhaltene p-[5-(4-Pentenyl)-2-pyridyl]benzonitril mit Klp. (N-I)−12°C konnte durch Abkühlen auf unter−70°C nicht kristallisiert werden.

In analoger Weise können folgende Verbindungen hergestellt werden :

p-[5-(3-Butenyl)-2-pyridyl]benzonitril,
p-[5-(3E-Pentenyl)-2-pyridyl]benzonitril,
p-[5-(4-(4-Pentenyl)phenyl)-2-pyridyl]benzonitril,
p-[5-(trans-4-(4-Pentenyl)cyclohexyl)-2-pyridyl]benzonitril.

Beispiel 3

a) 10 ml 3N Schwefelsäure wurden unter Rühren mit 1,8 g p-(5-Octyl-2-pyridyl)anilin versetzt. Das Gemisch wurde auf 5°C abgekühlt und dann bei 0-5°C innert ca. 3 Minuten tropfenweise mit einer Lösung von 0,442 g Natriumnitrit in 2 ml Wasser versetzt. Das erhaltene braune Gemisch wurde auf 100 ml Wasser gegossen und unter Rühren 30 Minuten zum Rückfluss erhitzt. Anschliessend wurde das Reaktionsgemisch auf

Raumtemperatur gekühlt, mit 7 ml 3N Natronlauge auf ca. pH 5 gestellt und einmal mit 200 ml Methylenchlorid und einmal mit 100 ml Methylenchlorid extrahiert. Die organischen Phasen wurden mit 150 ml verdünnter Kochsalzlösung gewaschen, über Natriumsulfat getrocknet und im Vakuum eingedampft. Der Rückstand (1,867 g) wurde an 250 g Kieselgel bei 0,4 bar mit Methylenchlorid und Methylenchlorid/Aceton chromatographisch getrennt. Umkristallisation des erhaltenen rotbraunen Oels in Diäthyläther/Hexan bei 0°C ergab 0,635 g p-(5-Octyl-2-pyridyl)phenol als beige Kristalle, Aufarbeitung der Mutterlauge ergab 0,355 g rohes p-(5-Octyl-2-pyridyl)phenol als braunes Oel.

b) Ein Gemisch von 0,355 g rohem p-(5-Octyl-2-pyridyl)phenol, 10 ml Aethanol, 0,260 g (S)-6-Methyloctylbromid, 0,172 g Kaliumcarbonat und einer Spatelspitze Natriumjodid wurde unter Rühren ca. 60 Stunden auf 90°C (Oelbadtemperatur) erwärmt. Anschliessend wurde die erhaltene braune Suspension abgekühlt und im Vakuum eingeengt. Der braune, ölige Rückstand wurde an 120 g Kieselgel bei 0,4 bar mit Methylenchlorid/ Aceton (Vol. 99 : 1) chromatographisch getrennt. Die erhaltene bräunliche Paste (0,367 g) wurde in ca. 5 ml Hexan gelöst, die Lösung filtriert und das Filtrat auf–25°C abgekühlt. Der Niederschlag wurde abgenutscht und bei 250°C/0,05 bar destilliert, wobei 0,122 g (S)-2-[p-(6-Methyloctyloxy)phenyl]-5-octylpyridin als leicht gelblicher, smektischer Flüssigkristall erhalten wurden. Einengen der Mutterlauge und anschliessende Destillation ergab weitere 0,164 g (S)-2-[p-(6-Methyloctyloxy)phenyl]-5-octylpyridin als leicht gelblicher, smektischer Flüssigkristall. Das Produkt (total 0,286 g) wurde mit Diäthyläther versetzt, abgedampft, 2 Stunden bei 80°C getrocknet und nochmals destilliert, wobei 0,245 g (S)-2-[p-(6-Methyloctyloxy)phenyl]-5-octylpyridin erhalten wurden ; Smp, (C-S) 36,1°C, Phasenübergang (S-$S_c^*$) 57°C, Klp. ($S_c^*$-I) 70,4°C.

In analoger Weise können folgende Verbindungen hergestellt werden :

2-[p-(Allyloxy)phenyl]-5-hexylpyridin,
2-[p-(2E-Butenyloxy)phenyl]-5-hexylpyridin,
2-[p-(3-Butenyloxy)phenyl]-5-hexylpyridin, Smp. (C-I) 43,5°C, Phasenübergang (S-I), 42,7°C,
2-[p-(4-Pentenyloxy)phenyl]-5-pentylpyridin,
2-[p-(4-Pentenyloxy)phenyl]-5-hexylpyridin,
5-[p-(Allyloxy)phenyl]-2-hexylpyridin,
5-[p-(2E-Butenyloxy)phenyl]-2-hexylpyridin,
5-[p-(3-Butenyloxy)phenyl]-2-hexylpyridin.

Beispiel 4

In einem Sulfierkolben mit mechanischem Rührer wird unter Argonbegasung eine Suspension von 6,64 g Methyltriphenylphosphoniumbromid in 80 ml t-Butylmethyläther bei –10°C innert 3 Minuten mit 2,12 g festem Kalium-t-butylat versetzt. Das Gemisch wird noch 1 Stunde bei Raumtemperatur gerührt, dann bei 0°C innert 5 Minuten mit einer Lösung von 3,61 g 4-[2-(p-Bromphenyl)-5-pyridyl]butyraldehyd in 20 ml t-Butylmethyläther versetzt und noch 15 Stunden bei Raumtemperatur gerührt. Anschliessend wird das Reaktionsgemisch dreimal in Diäthyläther/Wasser verteilt. Die organischen Extrakte werden zweimal mit Wasser gewaschen, über Magnesiumsulfat getrocknet, filtriert und eingedampft. Der Rückstand wird in Aethylacetat aufgenommen und dann das Gemisch mit Petroläther verdünnt und filtriert. Eindampfen des Filtrates und chromatographische Reinigung des Rückstandes an Kieselgel ergibt 2-(p-Bromphenyl)-5-(4-pentenyl)pyridin.

In analoger Weise können folgende Verbindungen hergestellt werden :

2-(p-Bromphenyl)-5-(3-butenyl)pyridin,
2-(p-Bromphenyl)-5-(3E-pentenyl)pyridin,
2-(p-Bromphenyl)-5-[4-(4-pentenyl)phenyl]pyridin,
2-(p-Bromphenyl)-5-[trans-4-(4-pentenyl)cyclohexyl]pyridin,
2-(p-Butylphenyl)-5-(4-pentenyl)pyridin,
2-(p-Butylphenyl)-5-(3E-pentenyl)pyridin,
2-(p-Pentylphenyl)-5-(4-pentenyl)pyridin,
5-Butyl-2-[p-(4-pentenyl)phenyl]pyridin,
5-Butyl-2-[p-(3E-pentenyl)phenyl]pyridin,
5-Pentyl-2-[p-(4-pentenyl)phenyl]pyridin,
2-(trans-4-Propylcyclohexyl)-5-(4-pentenyl)pyridin,
2-(trans-4-Propylcyclohexyl)-5-(3E-pentenyl)pyridin,
2-(trans-4-Butylcyclohexyl)-5-(4-pentenyl)pyridin,
2-(trans-4-Butylcyclohexyl)-5-(3E-pentenyl)pyridin,
2-(trans-4-Pentylcyclohexyl-5-(4-pentenyl)pyridin,

2-(trans-4-Pentylcyclohexyl-5-(3E-pentenyl)pyridin,
2-(trans-4-Heptylcyclohexyl-5-(4-pentenyl)pyridin,
2-(trans-4-Heptylcyclohexyl-5-(3E-pentenyl)pyridin.

Beispiel 5 (Ausgangsmaterialien)

a) Ein Gemisch von 0,248 g 2-(p-Bromphenyl)-5-methylpyridin, 0,196 g N-Bromsuccinimid, 20 mg Dibenzoylperoxid und 5 ml Tetrachlorkohlenstoff wurde unter Stickstoff-Begasung 2,5 Stunden zum Rückfluss erhitzt. Anschliessend wurde das Reaktionsgemisch auf Raumtemperatur abkühlen gelassen und genutscht. Der Rückstand wurde mit Tetrachlor-kohlenstoff nachgewaschen. Das Filtrat wurde im Vakuum eingeengt, wobei 0,360 g rohes 2-(p-Bromphenyl)-5-brommethyl-pyridin als bräunliche Kristalle erhalten wurden.

b) Ein Gemisch von 0,360 g rohem 2-(p-Bromphenyl)-5-brommethyl-pyridin, 0,5 ml Pyridin und 4 ml Benzol wurde 1 Stunde am Rückfluss gekocht. Anschliessend wurde die gelbe Suspension auf Raumtemperatur gekühlt und genutscht. Der Rückstand wurde mit Benzol gewaschen und im Vakuum getrocknet. Hierbei wurden 0,266 g N-[2-(p-Bromphenyl)-5-pyridyl]methyl-pyridiniumbromid als bräunliche Kristalle mit Smp. 264-268,5°C,(Zersetzung) erhalten.

c) Ein Gemisch von 0,266 g N-[2-(p-Bromphenyl)-5-pyridyl]methyl-pyridiniumbromid, 0,105 g 4-Nitroso-N,N-dimethyl-anilin und 2 ml Aethanol wurde unter Rühren mit 0,1 ml 3N Natronlauge versetzt und noch 1 Stunde bei Raumtemperatur gerührt. Anschliessend wurde das Reaktionsgemisch mit 2 ml 3N Salzsäure versetzt und zweimal mit je 50 ml Methylenchlorid extrahiert. Die Methylenchlorid-Phasen wurden mit 50 ml Wasser gewaschen, über Natriumsulfat getrocknet und im Vakuum getrocknet. Das Rohprodukt (120 mg grüne Kristalle) wurde an Kieselgel mit Methylenchlorid bei 0,4 bar chromatographisch gereinigt. Hierbei wurden 94 mg 2-(p-Bromphenyl)-5-pyridincarboxaldehyd als gelbe Kristalle erhalten ; Smp. 108,4-109,0°C nach Umkristallisation aus Methylenchlorid/Hexan.

In analoger Weise können folgende Verbindungen hergestellt werden :
5-(p-Bromphenyl)-2-pyridincarboxaldehyd,
4-[2-(p-Bromphenyl)-5-pyridyl]benzaldehyd,
4-[5-(p-Bromphenyl)-2-pyridyl]benzaldehyd,
2-(trans-4-Propylcyclohexyl)-5-pyridincarboxaldehyd,
2-(trans-4-Butylcyclohexyl)-5-pyridincarboxaldehyd,
2-(trans-4-Pentylcyclohexyl)-5-pyridincarboxaldehyd,
2-(trans-4-Heptylcyclohexyl)-5-pyridincarboxaldehyd.

Beispiel 6 (Ausgangsmaterialien)

a) Eine Lösung von 16,3 g p-[trans-4-(p-Anisyloxymethyl)cyclohexyl]benzoylchlorid in 50 ml Dichloräthan wird auf 7°C gekühlt und unter Stickstoff innert 5 Minuten portionenweise mit 6,4 g pulverisiertem Aluminiumchlorid versetzt. Dann wird bei 45°C während 17 Stunden Acetylengas in schwachem Strom durch das Reaktionsgemisch geleitet. Anschliessend wird das Reaktionsgemisch auf 100 ml Eiswasser gegossen, mit 50 ml 3N Salzsäure versetzt und 10 Minuten gerührt. Das Gemisch wird mit Diäthyläther extrahiert. Die organische Phase wird nacheinander mit Wasser, mit 5-prozentiger Kalilauge und mehrmals mit Wasser gewaschen, dann über Magnesiumsulfat getrocknet und eingedampft. Das erhaltene trans-4-(p-Anisyloxymethyl)-1-[p-(3-chloracryloyl)phenyl]cyclohexan wird an Kieselgel chromatographisch gereinigt.

b) Ein Gemisch von 3,15 g N-(1-Octenyl)piperidin, 2 ml Triäthylamin und 25 ml Diäthyläther wird unter Stickstoffbegasung tropfenweise mit einer Lösung von 6 g trans-4-(p-Anisyloxymethyl)-1-[p-(3-chloracryloyl)phenyl]cyclohexan in 8 ml Diäthyläther versetzt. Die Suspension wird 2,5 Stunden bei Raumtemperatur gerührt und dann zwischen Wasser und Diäthyläther verteilt. Die organische Phase wird mehrmals mit Wasser gewaschen, über Magnesiumsulfat getrocknet und eingedampft. Der Rückstand wird unter Kühlen portionenweise zu 25 ml 9-prozentiger wässriger Perchlorsäure gegeben. Das Gemisch wird 15 Minuten gerührt, dann mit 10 ml Aethanol versetzt und weitere 4,5 Stunden gerührt. Der entstandene Niederschlag an 5-Hexyl-2-[p-(trans-4-(p-anisyloxymethyl)cyclohexyl)phenyl]pyrylliumperchlorat wird abfiltriert, mit wenig eiskaltem Aethanol gewaschen und am Hochvakuum getrocknet.

c) Eine Lösung von 3,17 g 5-Hexyl-2-[p-(trans-4-(p-anisyloxymethyl)cyclohexyl)phenyl]pyrylliumperchlorat und 0,38 g Ammoniumacetat in 30 ml Eisessig wird 30 Minuten zum Rückfluss erhitzt. Danach wird das Reaktionsgemisch zwischen Methylenchlorid und Wasser verteilt. Die organische Phase wird nacheinander mit Wasser, gesättigter Natriumhydrogencarbonat-Lösung und zweimal mit Wasser gewaschen, über Magnesiumsulfat getrocknet und eingedampft. Das erhaltene 5-Hexyl-2-[p-(trans-4-(p-anisyloxymethyl)cyclohexyl)phenyl]pyridin wird an Kieselgel chromatographisch gereinigt.

d) Ein Gemisch von 0,485 g 5-Hexyl-2-[p-(trans-4-(p-anisyloxymethyl)cyclohexyl)phenyl]pyridin, 9,6 ml Acetonitril und 2,4 ml Wasser wird bei 0°C mit 1,25 g Cer-(IV)-ammoniumnitrat versetzt. Nach 10 Minuten wird das Reaktionsgemisch mit Wasser verdünnt und mit Diäthyläther extrahiert. Die organische Phase wird mehrmals mit Wasser gewaschen, dann über Magnesiumsulfat getrocknet und eingedampft. Chromatographische Reinigung des Rückstandes an Kieselgel ergibt 5-Hexyl-2-[p-(trans-4-(hydroxymethyl)cyclohexyl)phenyl]pyridin.

e) Eine Lösung von 0,94 ml Oxalylchlorid in 25 ml trockenem Methylenchlorid wird bei −60°C innert 10 Minuten tropfenweise mit einer Lösung von 1,56 ml Dimethylsulfoxid in 5 ml Methylenchlorid versetzt. 2 Minuten später wird das Reaktionsgemisch bei −60°C innert 10 Minuten tropfenweise mit einer Lösung von 3,51 g 5-Hexyl-2-[p-(trans-4-(hydroxymethyl)cyclohexyl)phenyl]pyridin in 10 ml Methylenchlorid versetzt und noch 15 Minuten bei −60°C gerührt. Danach wird das Reaktionsgemisch mit 7 ml Triäthylamin versetzt, auf Raumtemperatur aufwärmen gelassen und dann auf Wasser gegossen und mit Methylenchlorid extrahiert. Die organische Phase wird zweimal mit Wasser gewaschen, über Magnesiumsulfat getrocknet und eingedampft. Das erhaltene 5-Hexyl-2-[p-(trans-4-formylcyclohexyl)phenyl]pyridin wird an Kieselgel chromatographisch gereinigt.

Das als Ausgangsmaterial verwendete p-[trans-4-(p-Anisyloxymethyl)cyclohexyl]benzoylchlorid kann beispielsweise aus p-[trans-4-(Hydroxymethyl)cyclohexyl]benzonitril hergestellt werden durch Verätherung mit p-Hydroxyanisol in analoger Weise zu Tetr. Letters 26, 6291 (1985), Verseifung der Nitrilgruppe mit Kalilauge und Umsetzung der Carbonsäure mit Thionylchlorid zum Carbonsäurechlorid.

In analoger Weise können folgende Verbindungen hergestellt werden :

5-Octyl-2-[p-(trans-4-formylcyclohexyl)phenyl]pyridin,
5-Nonyl-2-[p-(trans-4-formylcyclohexyl)phenyl]pyridin,
5-Brom-2-[p-(trans-4-formylcyclohexyl)phenyl]pyridin,
5-Brom-2-[p-(trans-4-formylcyclohexyl)phenyl]pyridin.

Beispiel 7 (Ausgangsmaterialien)

a) In einem Sulfierkolben mit mechanischem Rührer wird unter Argonbegasung eine Suspension von 29,0 g Methoxymethyl-triphenylphosphoniumchlorid in 200 ml t-Butylmethyläther bei −10°C innert 3 Minuten mit 9,7 g Kalium-t-butylat versetzt. Die orange Suspension wird noch 1 Stunde bei ca. 0°C gerührt, dann bei−10°C innert 10 Minuten tropfenweise mit einer Lösung von 14,76 g 2-(p-Bromphenyl)-5-pyridincarboxaldehyd in 90 ml t-Butylmethyläther versetzt und noch 45 Minuten bei 0°C gerührt. Anschliessend wird das Reaktionsgemisch dreimal in Diäthyläther/Wasser verteilt. Die organischen Extrakte werden zweimal mit Wasser gewaschen, über Magnesiumsulfat getrocknet, filtriert und eingedampft. Zur Abtrennung von Triphenylphosphinoxid wird der Rückstand in Aethylacetat gelöst und die Lösung mit Petroläther verdünnt, filtriert und eingedampft. Chromatographische Reinigung des Rückstandes an Kieselgel ergibt 2-(p-Bromphenyl)-5-(2-methoxyvinyl)pyridin.

b) Eine Lösung von 12,4 g 2-(p-Bromphenyl)-5-(2-methoxyvinyl)pyridin in 200 ml Tetrahydrofuran/2N Salzsäure (Vol. 4 : 1) wird unter Rühren 1 Stunde zum Rückfluss erhitzt. Anschliessend wird das Reaktionsgemisch dreimal in Diäthyläther/Wasser verteilt. Die organischen Extrakte werden zweimal mit Wasser gewaschen, über Magnesiumsulfat getrocknet, filtriert und eingedampft, wobei [2-(p-Bromphenyl)-5-pyridyl]acetaldehyd als Rückstand erhalten wird.

c) [2-(p-Bromphenyl)-5-pyridyl]acetaldehyd wird in analoger Weise zu Absatz a) zu 2-(p-Bromphenyl)-5-(3-methoxy-2-propenyl)pyridin umgesetzt und dann dieses in analoger Weise zu Absatz b) zum 3-[2-(p-Bromphenyl)-5-pyridyl]propionaldehyd hydrolysiert.

d) 3-[2-(p-Bromphenyl)-5-pyridyl]propionaldehyd wird in analoger Weise zu Absatz a) zu 2-(p-Bromphenyl)-5-(4-methoxy-3-butenyl)pyridin umgesetzt und dann dieses in analoger Weise zu Absatz b) zum 4-[2-(p-Bromphenyl)-5-pyridyl]butyraldehyd hydrolysiert.

In analoger Weise können folgende Verbindungen hergestellt werden :

[5-(p-Bromphenyl)-2-pyridyl]acetaldehyd,
3-[5-(p-Bromphenyl)-2-pyridyl]propionaldehyd,
4-[5-(p-Bromphenyl)-2-pyridyl]butyraldehyd,
[4-(2-(p-Bromphenyl)-5-pyridyl)phenyl]acetaldehyd,
3-[4-(2-(p-Bromphenyl)-5-pyridyl)phenyl]propionaldehyd,
4-[4-(2-(p-Bromphenyl)-5-pyridyl)phenyl]butyraldehyd,
[4-(5-(p-Bromphenyl)-2-pyridyl)phenyl]acetaldehyd,
3-[4-(5-(p-Bromphenyl)-2-pyridyl)phenyl]propionaldehyd,

4-[4-(5-(p-Bromphenyl)-2-pyridyl)phenyl]butyraldehyd,
[trans-4-(2-(p-Bromphenyl)-5-pyridyl)cyclohexyl]acetaldehyd,
3-[trans-4-(2-(p-Bromphenyl)-5-pyridyl)cyclohexyl]propionaldehyd,
4-[trans-4-(2-(p-Bromphenyl)-5-pyridyl)cyclohexyl]butyralaldehyd,
[2-(p-Butylphenyl)-5-pyridyl]acetaldehyd,
3-[2-(p-Butylphenyl)-5-pyridyl]propionaldehyd,
4-[2-(p-Butylphenyl)-5-pyridyl]butyraldehyd,
[p-(5-Butyl-2-pyridyl)phenyl]acetaldehyd,
3-[p-(5-Butyl-2-pyridyl)phenyl]propionaldehyd,
4-[p-(5-Butyl-2-pyridyl)phenyl]butyraldehyd,
[trans-4-(p-(5-Hexyl-2-pyridyl)phenyl)cyclohexyl]acetaldehyd,
3-[trans-4-(p-(5-Hexyl-2-pyridyl)phenyl)cyclohexyl]propionaldehyd,
4-[trans-4-(p-(5-Hexyl-2-pyridyl)phenyl)cyclohexyl]butyraldehyd,
[trans-4-(p-(5-Octyl-2-pyridyl)phenyl)cyclohexyl]acetaldehyd,
3-[trans-4-(p-(5-Octyl-2-pyridyl)phenyl)cyclohexyl]propionaldehyd,
4-[trans-4-(p-(5-Octyl-2-pyridyl)phenyl)cyclohexyl]butyraldehyd,
[trans-4-[p-(5-(4-Pentenyl)-2-pyridyl)phenyl]cyclohexyl]acetaldehyd,
3-[trans-4-[p-(5-(4-Pentenyl)-2-pyridyl)phenyl]cyclohexyl]propionaldehyd,
4-[trans-4-[p-(5-(4-Pentenyl)-2-pyridyl)phenyl]cyclohexyl]butyraldehyd,
[2-(trans-4-Propylcyclohexyl)-5-pyridyl]acetaldehyd,
3-[2-(trans-4-Propylcyclohexyl)-5-pyridyl]propionaldehyd,
4-[2-(trans-4-Propylcyclohexyl)-5-pyridyl]butyraldehyd,
[2-(trans-4-Butylcyclohexyl)-5-pyridyl]acetaldehyd,
3-[2-(trans-4-Butylcyclohexyl)-5-pyridyl]propionaldehyd,
4-[2-(trans-4-Butylcyclohexyl)-5-pyridyl]butyraldehyd,
[2-(trans-4-Pentylcyclohexyl)-5-pyridyl]acetaldehyd,
3-[2-(trans-4-Pentylcyclohexyl)-5-pyridyl]propionaldehyd,
4-[2-(trans-4-Pentylcyclohexyl)-5-pyridyl]butyraldehyd,
[2-(trans-4-Heptylcyclohexyl)-5-pyridyl]acetaldehyd,
3-[2-(trans-4-Heptylcyclohexyl)-5-pyridyl]propionaldehyd,
4-[2-(trans-4-Heptylcyclohexyl)-5-pyridyl]butyraldehyd.

Durch weitere Kettenverlängerungsreaktionen sind die entsprechenden Valeraldehyde, Capronaldehyde, Heptaldehyde etc. erhältlich.

Beispiel 8 (Ausgangsmaterialien)

p-[trans-4-(p-Anisyloxy)cyclohexyl]benzoylchlorid wird nach der in Beispiel 6, Stufen a) bis d) beschriebenen Methode zu 5-Octyl-2-[p-trans-4-hydroxycyclohexyl)phenyl]pyridin umgesetzt.

In analoger Weise können folgende Verbindungen hergestellt werden :
5-Butyl-2-[p-(trans-4-hydroxycyclohexyl)phenyl]pyridin,
5-Pentyl-2-[p-(trans-4-hydroxycyclohexyl)phenyl]pyridin,
5-Hexyl-2-[p-(trans-4-hydroxycyclohexyl)phenyl]pyridin,
5-Heptyl-2-[p-(trans-4-hydroxycyclohexyl)phenyl]pyridin,
5-Nonyl-2-[p-(trans-4-hydroxycyclohexyl)phenyl]pyridin,
5-(4-Pentenyl)-2-[p-(trans-4-hydroxycyclohexyl)phenyl]pyridin,
5-(5-Hexenyl)-2-[p-(trans-4-hydroxycyclohexyl)phenyl]pyridin,
5-(7-Octenyl)-2-[p-(trans-4-hydroxycyclohexyl)phenyl]pyridin.

Beispiel 9

a) Eine Lösung von 12,2 g trans-4-Heptylcyclohexancarbonsäurechlorid in 50 ml Tetrachlorkohlenstoff wird unter Rühren auf 0°C gekühlt und innert 10 Minuten portionenweise mit 7 g pulverisiertem Aluminiumchlorid versetzt. Danach wird bei 0-3°C während 8 Stunden Acetylengas in das Reaktionsgemisch eingeleitet. Anschliessend wird das Reaktionsgemisch auf 120 g Eis und 60 ml 3N Salzsäure gegossen. Das Gemisch wird 30 Minuten gerührt und dann dreimal mit Diäthyläther extrahiert. Die organischen Phasen werden nacheinander mit Wasser, mit 5-prozentiger Kalilauge und dreimal mit Wasser gewaschen, dann über Natriumsulfat getrocknet, filtriert und eingeengt. Hierbei wird trans-4-Heptyl-1-(3-chloracryloyl)cyclohexan

(11,6 g) als bräunliche Flüssigkeit erhalten.

b) Ein Gemisch von 6 g N-(1-Propenyl)piperidin, 45 ml Diäthyläther und 4,36 ml Triäthylamin wird unter Rühren und Stickstoffbegasung bei 0°C innert 1 Stunde tropfenweise mit einer Lösung von 8,5 g trans-4-Heptyl-1-(3-chloracryloyl)cyclohexan in 20 ml Diäthyläther versetzt. Die Suspension wird noch 1 Stunde bei 0°C gerührt und dann mit 120 ml Wasser versetzt und dreimal mit Diäthyläther extrahiert. Die organischen Phasen werden zweimal mit Wasser gewaschen, über Natriumsulfat getrocknet, filtriert und eingeengt, wobei N-[5-(trans-4-Heptylcyclohexyl)-2-methyl-5-oxo-1,3-pentadienyl]piperidin erhalten wird.

c) Ein Gemisch von 25 ml 70-prozentiger Perchlorsäure, 25 ml Wasser und 25 ml Aethanol wird auf Raumtemperatur gekühlt, dann mit 8,7 g des erhaltenen N-[5-(trans-4-Heptylcyclohexyl)-2-methyl-5-oxo-1,3-pentadienyl]piperidins versetzt und noch 1,75 Stunden bei Raumtemperatur gerührt. Anschliessend wird das Reaktionsgemisch mit 160 ml Wasser versetzt und dreimal mit Methylenchlorid extrahiert. Die organischen Phasen werden zweimal mit Wasser gewaschen, über Natriumsulfat getrocknet und eingeengt. Hierbei wird 2-(trans-4-Heptylcyclohexyl)-5-methylpyrylliumperchlorat erhalten.

d) Ein Gemisch von 8,3 g 2-(trans-4-Heptylcyclohexyl)-5-methylpyrylliumperchlorat, 116 ml Eisessig und 3,57 g Ammoniumacetat wird 30 Minuten zum Rückfluss erhitzt. Danach wird das Reaktionsgemisch auf Raumtemperatur abgekühlt, mit 180 ml Wasser versetzt und zweimal mit Methylenchlorid extrahiert. Die organischen Phasen werden nacheinander mit Wasser, mit 10-prozentiger wässriger Natriumcarbonat-Lösung und zweimal mit Wasser gewaschen, dann über Natriumsulfat getrocknet und filtriert. Einengen des Filtrates und chromatographische Reinigung ergibt 2-(trans-4-Heptylcyclohexyl)-5-methylpyridin.

Beispiel 10 (nematisches Gemisch)

14 Gew-.% p-(trans-4-Propylcyclohexyl)benzonitril,
19 " p-[trans-4-(4-Pentenyl)cyclohexyl]benzonitril,
11 " 4'-[trans-4-(4-Pentenyl)cyclohexyl]-4-biphenylcarbonitril,
14 " p-(5-Butyl-2-pyrimidinyl)benzonitril,
4 " p-(5-Pentyl-2-pyrimidinyl)benzonitril,
6 " p-[5-(trans-4-Aethylcyclohexyl)-2-pyrimidinyl]benzonitril,
10 " p-[trans-5-(4-Pentenyl)-m-dioxan-2-yl]phenylisothiocyanat,
7 " p-(4-Pentenyl)benzoesäure-4-cyano-3-fluorphenylester,
7 " trans-4-Propylcyclohexancarbonsäure-p-[trans-4(3E-pentenyl)cyclohexylphenylester,
8 " p-(5-Pentyl-2-pyridyl)phenylisothiocyanat ;
Smp. (C-N) $<-20$°C, Klp. (N-I) 61°C ; $p_o$ = 0,137, $\Delta n$ = 0,165.

## Ansprüche

1. Verbindungen der allgemeinen Formel

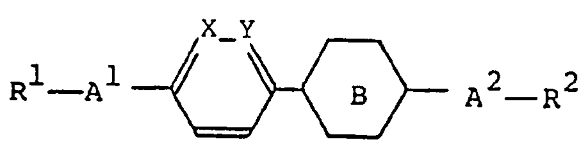

I

X für CH und Y für N steht oder X für N und Y für CH steht ; Ring B trans-1,4-Cyclohexylen oder gegebenenfalls mit Halogen oder Methyl substituiertes 1,4-Phenylen darstellt ; $A^1$ und $A^2$ einfache Kovalenzbindungen bedeuten oder eine der Gruppen $A^1$ und $A^2$ auch trans-1,4-Cyclohexylen oder gegebenenfalls mit Halogen oder Methyl substituiertes 1,4-Phenylen bedeutet ; $R^1$ Alkyl oder Alkenyl bezeichnet, in welchen gegebenenfalls eine oder zwei nicht benachbarte $CH_2$-Gruppen durch Sauerstoff ersetzt sind, und $R^2$ Alkenyl bedeutet, in welchem gegebenenfalls eine oder zwei nicht benachbarte $CH_2$-Gruppen durch Sauerstoff ersetzt sind, oder $R^2$ an einem Benzolring auch –NCS bedeutet ; oder $R^1$ Alkenyl bezeichnet, in welchem gegebenenfalls eine oder zwei nicht benachbarte $CH_2$-Gruppen durch Sauerstoff ersetzt sind, und $R^2$ Alkyl bedeutet, in welchem gegebenenfalls eine oder zwei nicht benachbarte $CH_2$-Gruppen durch Sauerstoff ersetzt sind, oder $R^2$ an einem Benzolring auch Cyano oder Halogen bedeutet ; mit der Massgabe, dass $R^1$ und $R^2$ höchstens je 18 Kohlenstoffatome

aufweisen.

2. Verbindungen nach Anspruch 1 dadurch gekennzeichnet, dass $R^2$ –NCS oder Cyano bedeutet.

3. Verbindungen der allgemeinen Formel

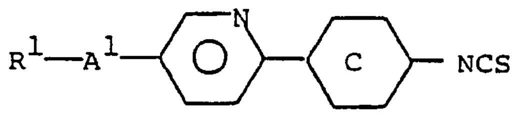

Ia

worin $R^1$ die in Anspruch 1 gegebene Bedeutung hat; $A^1$ eine einfache Kovalenzbindung, trans-1,4-Cyclohexylen oder gegebenenfalls mit Halogen oder Methyl substituiertes 1,4-Phenylen bedeutet; und Ring C gegebenenfalls mit Halogen oder Methyl substituiertes 1,4-Phenylen darstellt.

4. Verbindungen nach Anspruch 3, dadurch gekennzeichnet, dass $R^1$ Alkyl oder Alkenyl mit höchstens 18 Kohlenstoffatomen bezeichnet, in welchen gegebenenfalls eine oder zwei $CH_2$-Gruppen durch Sauerstoff ersetzt sind.

5. Verbindungen nach Anspruch 4, dadurch gekennzeichnet, dass $R^1$ geradkettiges Alkyl, Alkoxy, Alkenyl oder Alkenyloxy mit 1 bis 12 Kohlenstoffatomen bedeutet.

6. Verbindungen nach einem der Ansprüche 3 bis 5, dadurch gekennzeichnet, dass $R^1$ geradkettiges Alkyl, Alkoxy, Alkenyl oder Alkenyloxy mit 1 bis 7 Kohlenstoffatomen bezeichnet, $A^1$ eine einfache Kovalenzbindung darstellt, und Ring C 1,4-Phenylen bedeutet.

7. Verbindungen nach Anspruch 1, dadurch gekennzeichnet, dass $R^2$ eine Alkyl- oder Alkenylgruppe bedeutet, in welcher gegebenenfalls eine oder zwei nicht benachbarte $CH_2$-Gruppen durch ersetzt sind.

8. Optisch aktive Verbindungen nach Anspruch 7, dadurch gekennzeichnet, dass $R^1$ und/oder $R^2$ ein chirales Kohlenstoffatom aufweist.

9. Verbindungen der allgemeinen Formel

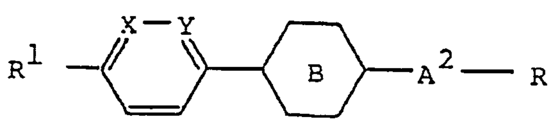

Ii

worin $R^1$, X, Y und Ring B die in Anspruch 1 gegebenen Bedeutungen haben; $A^2$ eine einfache Kovalenzbindung, trans-1,4-Cyclohexylen oder gegebenenfalls mit Halogen oder Methyl substituiertes 1,4-Phenylen bedeutet; und R eine Alkyl- oder Alkenylgruppe bezeichnet, in welcher gegebenenfalls eine oder zwei nicht benachbarte $CH_2$-Gruppen durch –O– ersetzt sind.

10. Verbindungen nach Anspruch 9, dadurch gekennzeichnet, dass Ring B gegebenenfalls mit Halogen oder Methyl substituiertes 1,4-Phenylen darstellt.

11. Optisch aktive Verbindungen nach einem der Ansprüche 8 bis 10, dadurch gekennzeichnet, dass $R^1$ und/oder $R^2$ bzw. $R^1$ und/oder R eine Gruppe der allgemeinen Formeln

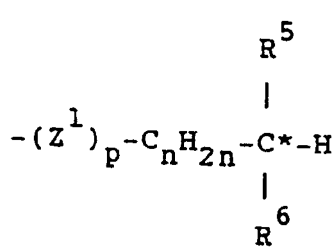

IV

oder

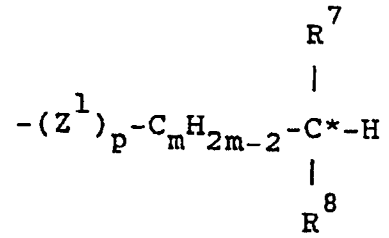

V

worin m, n und p ganze Zahlen bedeuten und p für 0 oder 1, n für 0-6 und m für 2-6 stehen ; $R^5$ Alkyl und $R^6$ Alkoxy, Alkenyl, Alkenyloxy oder von $R^5$ verschiedenes Alkyl bedeuten ; oder $R^5$ Alkenyl und $R^6$ Alkoxy bedeuten ; $R^7$ Alkyl und $R^8$ Alkoxy oder von $R^7$ verschiedenes Alkyl bezeichnen ; und $Z^1$ die Gruppe $-CH_2$ -oder $-O-$ darstellt, bezeichnet.

12. Optisch aktive Verbindungen nach Anspruch 11, dadurch gekennzeichnet, dass $Z^1$ Sauerstoff bedeutet.

13. Optisch aktive Verbindungen nach Anspruch 11 oder 12, dadurch gekennzeichnet, dass $R^5$ und $R^7$ Methyl bedeuten und $R^6$ und $R^8$ von Methyl verschiedenes Alkyl bezeichnen.

14. Optisch aktive Verbindungen nach einem der Ansprüche 8 bis 13, dadurch gekennzeichnet, dass die Reste $R^1$ und $R^2$ bzw. $R^1$ und R höchstens je 18 Kohlenstoffatome aufweisen und die Summe der Kohlenstoffatome in beiden Resten zusammen im Falle bicyclischer Verbindungen mindestens 10 und im Falle tricyclischer Verbindungen mindestens 8 beträgt.

15. Verbindungen der allgemeinen Formel

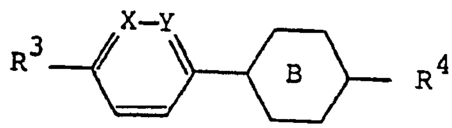

Ib

worin einer der Reste $R^3$ und $R^4$ eine Alkenylgruppe bezeichnet, in welcher gegebenenfalls eine oder zwei nicht benachbarte $CH_2$-Gruppen durch –O– elsetzt sind ; der andere der Reste $R^3$ und $R^4$ eine Alkyl- oder Alkenylgruppe bedeutet, in welcher gegebenenfalls eine oder zwei nicht benachbarte $CH_2$-Gruppen durch –O– ersetzt sind ; und X, Y und Ring B die in Anspruch 1 gegebenen Bedeutungen haben.

16. Verbindungen nach Anspruch 15, dadurlch gekennzeichnet, dass $R^3$ und $R^4$ geradkettige Reste mit je 1-12, vorzugsweise je 1-7 Kohlenstoffatomen bedeuten.

17. Verbindungen nach einem der Ansprüche 1 bis 3 und 7 bis 16, dadurch gekennzeichnet, dass $R^1$ und $R^3$ Alkyl, Alkoxy, Alkoxyalkyl, Alkoxyalkoxy, Alkenyl, Alkenyloxy, Alkoxyalkenyl, Alkoxyalkenyloxy oder Alkenyloxyalkyl mit höchstens 18 Kohlenstoffatomen bedeuten.

18. Verbindungen nach Anspruch 17, dadurch gekennzeichnet, dass $R^1$ und $R^3$ Alkyl, Alkoxy, 1E-Alkenyl, 3E-Alkenyl, 4-Alkenyl, 2E-Alkenyloxy oder 3-Alkenyloxy bedeuten.

19. Verbindungen nach einem der Ansprüche 1 und 7 bis 18, dadurch gekennzeichnet, dass $R^2$, R und $R^4$ Alkyl, Alkoxy, Alkoxyalkyl, Alkoxyalkoxy, Alkenyl, Alkenyloxy, Alkoxyalkenyl, Alkoxyalkenyl oder Alkenyloxyalkyl mit höchstens 18 Kohlenstoffatomen bedeuten.

20. Verbindungen nach Anspruch 19, dadurch gekennzeichnet, dass $R^2$, R und $R^4$ Alkyl, Alkoxy, 1E-Alkenyl, 3E-Alkenyl, 4-Alkenyl, 2E-Alkenyloxy oder 3-Alkenyloxy bedeuten.

21. Verbindungen nach einem der Ansprüche 1, 2 und 9 bis 20, dadurch gekennzeichnet, dass X für CH und Y für N steht.

22. Flüssigkristallines Gemisch mit mindestens 2 Komponenten, dadurch gekennzeichnet, dass mindestens eine Komponente eine Verbindung gemäss Ansprüchen 1 bis 21 ist.

23. Verwendung der in den Ansprüchen 1 bis 21 definierten Verbindungen I für elektro-optische Zwecke.

**Claims**

1. Compounds of the general formula

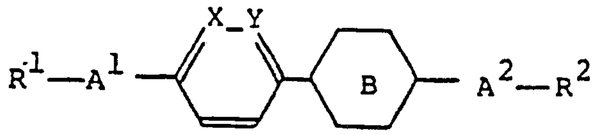

I

wherein X stands for CH and Y stands for N or X stands for N and Y stands for CH ; ring B represents trans-1,4-cyclohexylene or 1,4-phenylene optionally substituted with halogen or methyl ; $A^1$ and $A^2$ signify single covalent bonds or one of the groups $A^1$ and $A^2$ also signifies trans-1,4-cyclohexylene or 1,4-phenylene optionally substituted with halogen or methyl ; $R^1$ denotes alkyl or alkenyl in which optionally one $CH_2$ group or two non-adjacent $CH_2$ groups is/are replaced by oxygen and $R^2$ signifies alkenyl in which optionally one $CH_2$ group or two non-adjacent $CH_2$ groups is/are replaced by oxygen or $R^2$ on a benzene ring also signifies –NCS; or $R^1$ denotes alkenyl in which optionally one $CH_2$ group or two non-adjacent $CH_2$ groups is/are replaced by oxygen and $R^2$ signifies alkyl in which optionally one $CH_2$ group or two non-adjacent $CH_2$ groups is/are replaced by oxygen, or $R^2$ on a benzene ring also signifies cyano or halogen ; with the proviso that $R^1$ and $R^2$ each have a maximum of 18 carbon atoms.

2. Compounds according to claim 1, characterized in that $R^2$ signifies –NCS or cyano.

3. Compounds of the general formula

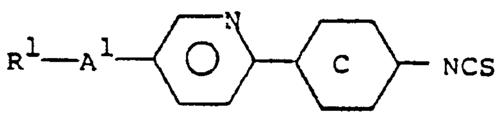

Ia

wherein $R^1$ has the significance given in claim 1 ; $A^1$ signifies a single covalent bond, trans-1,4-cyclohexylene or 1,4-phenylene optionally substituted with halogen or methyl ; and ring C represents 1,4-phenylene optionally substituted with halogen or methyl.

4. Compounds according to claim 3, characterized in that $R^1$ denotes alkyl or alkenyl with a maximum of 18 carbon atoms in which optionally one $CH_2$ group or two non-adjacent $CH_2$ groups is/are replaced by oxygen.

5. Compounds according to claim 4, characterized in that $R^1$ signifies straight-chain alkyl, alkoxy, alkenyl or alkenyloxy with 1 to 12 carbon atoms.

6. Compounds according to any one of claims 3 to 5, characterized in that $R^1$ denotes straight-chain alkyl, alkoxy, alkenyl or alkenyloxy with 1 to 7 carbon atoms, $A^1$ represents a single covalent bond and ring C signifies 1,4-phenylene.

7. Compound according to claim 1, characterized in that $R^2$ signifies an alkyl or alkenyl group in which optionally one $CH_2$ group or two non-adjacent $CH_2$ groups is/are replaced by –O–.

8. Optically active compounds according to claim 7, characterized in that $R^1$ and/or $R^2$ has a chiral carbon atom.

9. Compounds of the general formula

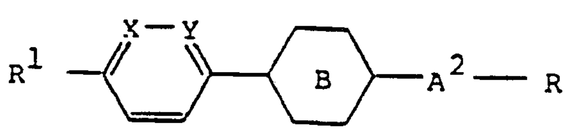

Ii

wherein $R^1$, X, Y and ring B have the significances given in claim 1 ; $A^2$ signifies a single covalent bond, trans-1,4-cyclohexylene or 1,4-phenylene optionally substituted with halogen or methyl ; and R denotes an alkyl or alkenyl group in which optionally one $CH_2$ group or two non-adjacent $CH_2$ groups is/are replaced by –O–.

10. Compounds according to claim 9, characterized in that ring B represents 1,4-phenylene optionally substituted with halogen or methyl.

11. Optically active compounds according to any one of claims 8 to 10, characterized in that $R^1$ and/or $R^2$ or $R^1$ and/or R denotes a group of the general formula

$$-(Z^1)_p-C_nH_{2n}-\overset{\displaystyle R^5}{\underset{\displaystyle R^6}{C^*}}-H \qquad \text{IV}$$

or

$$-(Z^1)_p-C_mH_{2m-2}-\overset{\displaystyle R^7}{\underset{\displaystyle R^8}{C^*}}-H \qquad \text{V}$$

wherein m, n and p signify whole numbers and p stands for 0 or 1, n stands for 0-6 and m stands for 2-6 ; $R^5$ signifies alkyl and $R^6$ signifies alkoxy, alkenyl, alkenyloxy or alkyl different from $R^5$ ; or $R^5$ signifies alkenyl and $R^6$ signifies alkoxy ; $R^7$ denotes alkyl and $R^8$ denotes alkoxy or alkyl different from $R^7$ ; and $Z^1$ represents the group $-CH_2-$ or $-O-$.

12. Optically active compounds according to claim 11, characterized in that $Z^1$ signifies oxygen.

13. Optically active compounds according to claim 11 or 12, characterized in that $R^5$ and $R^7$ signify methyl and $R^6$ and $R^8$ denote alkyl different from methyl.

14. Optically active compounds according to any one of claims 8 to 13, characterized in that the residues $R^1$ and $R^2$ or $R^1$ and R each have a maximum of 18 carbon atoms and the sum of the carbon atoms in the two residues together amounts to at least 10 in the case of bicyclic compounds and to at least 8 in the case of tricyclic compounds.

15. Compounds of the general formula

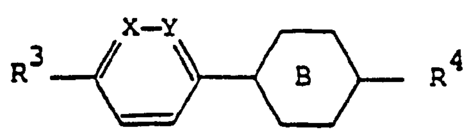

Ib

wherein one of the residue $R^3$ and $R^4$ denotes an alkenyl group in which optionally one $CH_2$ group or two non-adjacent $CH_2$ groups is/are replaced by –O– ; the other of the residues $R^3$ and $R^4$ denotes an alkyl or alkenyl group in which optionally one $CH_2$ group or two non-adjacent $CH_2$ groups is/are replaced by –O– ; and X, Y and ring B have the significance given in claim 1.

16. Compounds according to claim 15, characterized in that $R^3$ and $R^4$ signify straight-chain residues each having 1-12, preferably 1-7, carbon atoms.

17. Compounds according to any one of claims 1 to 3 and 7 to 16, characterized in that $R^1$ and $R^3$ signify alkyl, alkoxy, alkoxyalkyl, alkoxyalkoxy, alkenyl, alkenyloxy, alkoxyalkenyl, alkoxyalkenyoxy or alkenyloxyalkyl with a maximum of 18 carbon atoms.

18. Compounds according to claim 17, characterized in that $R^1$ and $R^3$ signify alkyl, alkoxy, 1E-alkenyl, 3E-alkenyl, 4-alkenyl, 2E-alkenyloxy or 3-alkenyloxy.

19. Compounds according to any one of claims 1 and 7 to 18, characterized in that $R^2$, R and $R^4$ signify alkyl, alkoxy, alkoxyalkyl, alkoxyalkoxy, alkenyl, alkenyloxy, alkoxyalkenyl, alkoxyalkenyloxy or alkenyloxyalkyl with a maximum of 18 carbon atoms.

20. Compounds according to claim 19, characterized in that $R^2$, R and $R^4$ signify alkyl, alkoxy, 1E-alkenyl, 3E-alkenyl, 4-alkenyl, 2E-alkenyloxy or 3-alkenyloxy.

21. Compounds according to any one of claims 1, 2 and 9 to 20, characterized in that X stands for CH and Y stands for N.

22. A liquid crystalline mixture with at least 2 components, characterized in that at least one component is a compound in accordance with claims 1 to 21.

23. The use of the compounds I defined in claims 1 to 21 for electro-optical purposes.

**Revendications**

1. Composés de formule générale

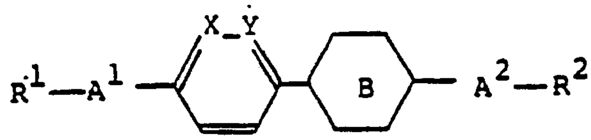

I

dans laquelle

X représente CH et Y représente N ou bien X représente N et Y représente CH ; le noyau B est un noyau trans-1,4-cyclohexylène ou un noyau 1,4-phénylène éventuellement substitué par des halogènes ou des groupes méthyle ; $A^1$ et $A^2$ représentent des liaisons covalentes simples, l'un des symboles $A^1$ et $A^2$ pouvant également représenter un noyau trans-1,4-cyclohexylène ou un noyau 1,4-phénylène éventuellement substitué par des halogènes ou des groupes méthyle ; $R^1$ représente un groupe alkyle ou alcényle dans lequel, le cas échéant, un ou deux groupes $CH_2$ non voisins sont remplacés par l'oxygène, et $R^2$ représente un groupe alcényle dans lequel, le cas échéant, un ou deux groupes $CH_2$ non voisins sont remplacés par l'oxygène, ou bien $R^2$, sur un noyau benzénique, peut également représenter –NCS ; ou bien $R^1$ représente un groupe alcényle dans lequel, le cas échéant, un ou deux groupes $CH_2$ non voisins sont remplacés par l'oxygène, et $R^2$ représente un groupe alkyle dans lequel, le cas échéant, un ou deux groupes $CH_2$ non voisins sont remplacés par l'oxygène, ou bien $R^2$, sur un noyau benzénique, peut également représenter un groupe cyano ou un halogène ; sous réserve que $R^1$ et $R^2$ contiennent chacun au maximum 18 atomes de carbone.

2. Composés selon la revendication 1, caractérisés en ce que $R^2$ représente un groupe –NCS ou cyano.

3. Composés de formule générale

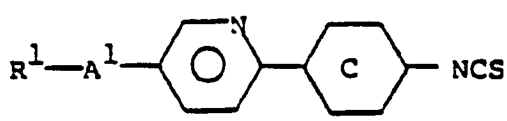

Ia

dans laquelle $R^1$ a les significations indiquées dans la revendication 1 ; $A^1$ représente une liaison covalente simple, un noyau trans-1,4-cyclohexylène ou un noyau 1,4-phénylène éventuellement substitué par des halogènes ou des groupes méthyle ; et le noyau C est un noyau 1,4-phénylène éventuellement substitué par des halogènes ou des groupes méthyle.

4. Composés selon la revendication 3, caractérisés en ce que $R^1$ représente un groupe alkyle ou alcényle à 18 atomes de carbone au maximum et dans lequel, le cas échéant, un ou deux groupes $CH_2$ sont remplacés par l'oxygène.

5. Composés selon la revendication 4, caractérisés en ce que $R^1$ représente un groupe alkyle, alcoxy, alcényle ou alcényloxy à chaîne droite en C1-C12.

6. Composés selon l'une des revendications 3 à 5, caractérisés en ce que $R^1$ représente un groupe alkyle, alcoxy, alcényle ou alcényloxy à chaîne droite en C1-C7, $A^1$ représente une liaison covalente simple et le noyau C est un noyau 1,4-phénylène.

7. Composés selon la revendication 1, caractérisés en ce que $R^2$ représente un groupe alkyle ou alcényle dans lequel, le cas échéant, un ou deux groupes CH2 non voisins sont remplacés par –O–.

8. Composés selon la revendication 7, possédant l'activité optique, caractérisés en ce que $R^1$ et/ou $R^2$ contiennent un atome de carbone chiral.

9. Composés de formule générale

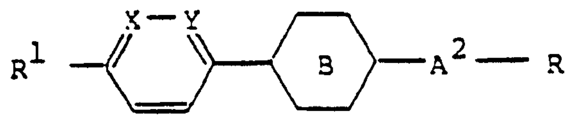 Ii

dans laquelle $R^1$, X, Y et le noyau B sont tels que décrits dans la revendication 1 ; $A^2$ représente une liaison covalente simple, un noyau trans-1,4-cyclohexylène ou un noyau 1,4-phénylène éventuellement substitué par des halogènes ou des groupes méthyle ; et R représente un groupe alkyle ou alcényle dans lequel, le cas échéant, un ou deux groupes $CH_2$ non voisins sont remplacés par –O–.

10. Composés selon la revendication 9, caractérisés en ce que le noyau B est un noyau 1,4-phénylène éventuellement substitué par des halogènes ou des groupes méthyle.

11. Composés selon l'une des revendications 8 à 10, possédant l'activité optique, caractérisés en ce que $R^1$ et/ou $R^2$ ou respectivement $R^1$ et/ou R représentent un groupe de formule générale

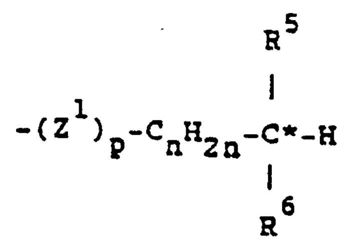 IV

ou

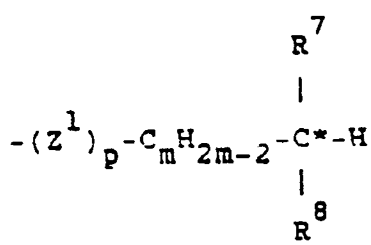 V

dans lesquelles m, n et p sont des nombres entiers et p est égal à 0 ou 1, n a une valeur de 0 à 6 et m une valeur de 2 à 6 ; $R^5$ représente un groupe alkyle et $R^6$ un groupe alcoxy, alcényle, alcényloxy ou un groupe alkyle autre que celui représenté par $R^5$ ; ou bien $R^5$ représente un groupe alcényle et $R^6$ un groupe alcoxy ; $R^7$ représente un groupe alkyle et $R^8$ un groupe alcoxy ou un groupe alkyle autre que celui représenté par $R^7$; et $Z^1$ représente $-CH_2-$ ou –O–.

12. Composés selon la revendication 11, possédant l'activité optique, caractérisés en ce que $Z^1$ représente l'oxygène.

13. Composés selon la revendication 11 ou 12, possédant l'activité optique, caractérisés en ce que $R^5$ et $R^7$ représentent des groupes méthyle et $R^6$ et $R^8$ des groupes alkyle autres que des groupes méthyle.

14. Composés selon l'une des revendications 8 à 13, possédant l'activité optique, caractérisés en ce que les groupes $R^1$ et $R^2$ ou respectivement $R^1$ et R contiennent au maximum chacun 18 atomes de carbone et la somme des atomes de carbone des deux groupes est d'au moins 10 dans le cas de composés bicycliques et d'au moins 8 dans le cas de composés tricycliques.

15. Composés de formule générale

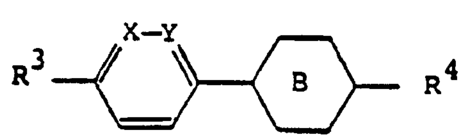 Ib

dans laquelle l'un des symboles $R^3$ et $R^4$ représente un groupe alcényle dans lequel, le cas échéant, un ou deux groupes $CH_2$ non voisins sont remplacés par –O– ; l'autre de ces symboles $R^3$ et $R^4$ représente un groupe alkyle ou alcényle dans lequel, le cas échéant, un ou deux groupes $CH_2$ non voisins sont remplacés par –O–; et X, Y et le noyau B sont tels qu'indiqués dans la revendication 1.

16. Composés selon la revendication 15, caractérisés en ce que $R^3$ et $R^4$ représentent des groupes à chaîne droite contenant chacun 1 à 12, de préférence 1 à 7 atomes de carbone.

17. Composés selon l'une des revendications 1 à 3 et 7 à 16, caractérisés en ce que $R^1$ et $R^3$ représentent des groupes alkyle, alcoxy, alcoxyalkyle, alcoxyalcoxy, alcényle, alcényloxy, alcoxyalcényle, alcoxyalcényloxy ou alcényloxyalkyle contenant au maximum 18 atomes de carbone.

18. Composés selon la revendication 17, caractérisés en ce que $R^1$ et $R^3$ représentent des groupes alkyle, alcoxy, 1E-alcényle, 3E-alcényle, 4-alcényle, 2E-alcényloxy ou 3-alcényloxy.

19. Composés selon l'une des revendications 1 et 7 à 18, caractérisés en ce que $R^2$, R et $R^4$ représentent des groupes alkyle, alcoxy, alcoxyalkyle, alcoxyalcoxy, alcényle, alcényloxy, alcoxyalcényle, alcoxyalcényle ou alcényloxyalkyle contenant au maximum 18 atomes de carbone.

20. Composés selon la revendication 19, caractérisés en ce que $R^2$, R et $R^4$ représentent des groupes alkyle, alcoxy, 1E-alcényle, 3E-alcényle, 4-alcényle, 2E-alcényloxy ou 3-alcényloxy.

21. Composés selon l'une des revendications 1, 2 et 9 à 20, caractérisés en ce que X représente CH et Y représente N.

22. Mélange à cristaux liquides à au moins deux composants, caractérisés en ce qu'au moins un composant consiste en un composé selon l'une des revendications 1 à 21.

23. Utilisation des composés I définis dans les revendications 1 à 21 dans des applications électro-optiques.